# EUROPEAN PATENT APPLICATION

(11) **EP 0 630 969 A1**
(43) Date of publication of application: **28.12.1994**
(21) Application number: 93401585.0
(22) Date of filing: 21.06.1993
(51) Int. Cl.: C12N 15/74, C12N 15/31, C07K 13/00, C12N 1/21, C12P 21/02, A23L 1/317, A23C 19/032

(54) **Vectors containing nucleotide sequences of a plasmid isolated from Lactobacillus curvatus, proteins coded by the genome of these vectors, and the use of such vectors in processes for the production of polypeptides**

(71) Applicant: COMMUNAUTE ECONOMIQUE EUROPEENNE (CEE), L-2920 Luxembourg (LU)
(72) Inventor: Klein, Jürgen Robert, W-6751 Weilerbach (DE); Henrich, Bernhard, W-6751 Geiselberg (DE); Plapp, Roland, W-6750 Kaiserslautern (DE)
(74) Representative: Grosset-Fournier, Chantal Catherine

(57) **Abstract**

The invention relates to nucleic acids comprising all or part of the nucleotide sequence of a plasmid isolated from *Lactobacillus curvatus*.

The invention relates more particularly to the use of such nucleic acids as vectors for transformation of host cells, for example in fermentation processes.

SEQUENCE LISTING

(1) GENERAL INFORMATION:

(i) APPLICANT:
(A) NAME: COMMUNAUTE ECONOMIQUE EUROPEENNE
(B) STREET: BATIMENT JEAN MONNET - PLATEAU DU KIRCHBERG
(C) CITY: LUXEMBOURG
(D) STATE: LUXEMBOURG
(E) COUNTRY: LUXEMBOURG
(F) POSTAL CODE (ZIP): L2920

(ii) TITLE OF INVENTION: VECTORS CONTAINING NUCLEOTIDE SEQUENCES OF A
PLASMID ISOLATED FROM LACTOBACILLUS CURVATUS, PROTEINS
CODED BY THE GENOME OF THESE VECTORS, AND THE USE OF SUCH VECTORS IN
PROCESSES FOR THE PRODUCTION OF

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 2489 base pairs
(B) TYPE: nucleic acid
(C) STRANDEDNESS: double
(D) TOPOLOGY: circular

(ii) MOLECULE TYPE: DNA (genomic)

(ix) FEATURE:
(A) NAME/KEY: CDS
(B) LOCATION: 1049..1216

(ix) FEATURE:
(A) NAME/KEY: CDS
(B) LOCATION: 1303..1965

(ix) FEATURE:
(A) NAME/KEY: CDS
(B) LOCATION: 2000..2437

(xi) SEQUENCE DESCRIPTION: SEQ ID NO: 1:

GTCGACTTCT GATACAAAAT TTTAGGTTTT TAGCATGGCA CAACTTCATG TTGTTTATAA 60

GTGCGCATTT ATTTTGCTTT ACAAAATTTA CGGACAGTTT AATTTAGTGA TTATTTATAA 120

AACCAGTAAT AAATCCAAAA AGCATAGGTA AAATAAGACC AAGGCATAAA AGGATAAAGC 180

CCAAGCGACA CCATATATTT AACTTTGGGT ATAAATCCTT GAAATTAAAC AACATAATTA 240

TCAGACCTAA AAAAGCAATA AAATATCCTG ATGTATAAAT CATTTTGAAT CTCCTTTCAA 300

CCAGTCATGT ACTTTTTACT CACCTATTAT GCTCATAAAC GTTGATATAA AGCCAACCTC 360

ATTTCTCCAA ATAAATTGGA GTTGTGCTAA AACACTTAAA CCTGTATCAG ATTTTGCTTT 420

GCTCAAACAA AACTGACTTG CGTCAGTTGG AATCTTCAAA ACCAATAAAG TCCAGTCGCC 480

AACTCCTTCA GACTTTATTA ACTTTGAAGA TTGCTTTTAA ATGCCTCTAA TTTGCTCTCT 540

AAGCCATTTT AGCTGTTACC CGTATAATTT ACTGTCCGTC AACGGTAAAT CGACGTAGAA 600

CGGCTTTTAG CGTCTGGGAG GCTTTAAGGA GTTGACGGAC TCACTAGGCC AAGACACTTT 660

TGCGCATGCA AAGAAAAGCA CACCTGCTTT TTTGCCTGCC TCACGGCGAG TGCGGGGTGA 720

GTTTGAGCGG GAGTCCGTCA TCTATGGGGT CAAGCTGACA CAGCTTGCAG GTTTGGGCAC 780

CATGGTTTTA TTCGTGCGGG ATAGAAATTT GGAAATCAGG GGGGCGAGGG AGCGAATTTT 840

GCGACCGTAC TACGACCCCC CTTTTAAGTG CCGAGTGCCA AATCGGTCAA CAAACCCTGA 900

CAAACCCATA CACACCAAGT GTTACAGCGA TCAACAAAAA GTCAACAACC CGCCAACAAA 960

ATTTTGGAAA TATAATGATA AAAAGGTAGT TGAATATATA TATATAAAGT TGTATAATGT 1020

TCTTATGAAC ATAAAAGGAG TGTTATAA ATG GCT AGA GAG AAA TCA ACG ATA 1072
Met Ala Arg Glu Lys Ser Thr Ile
1               5

GAA TAC CAA AAT GTA TCA ATG CGT ATT CCC AAA GAA TTA TAT TCT GAA 1120
Glu Tyr Gln Asn Val Ser Met Arg Ile Pro Lys Glu Leu Tyr Ser Glu
    10                  15                  20

TAT AAA AAA GTT TTG GCT AAA ACT GGA TCT ATT GTT ACT TAT GAC GTT 1168
Tyr Lys Lys Val Leu Ala Lys Thr Gly Ser Ile Val Thr Tyr Asp Val
25                  30                  35                  40

CGA AAT TAT ATG AAA AAA GTT GTT GAA GAA GAT AAG AAA GGA CAA AAA 1216
Arg Asn Tyr Met Lys Lys Val Val Glu Glu Asp Lys Lys Gly Gln Lys
                45                  50                  55

TGAGTATAAA AAAAGAGCGC CCTCTTCGAA AAGTCGCTCA TGTTTTTATA ATGTTTTTAC 1276

TAATTTTTGT AAGGTAATTA TATCAA ATG AGT AAA GAA AAA GCT AGA TAC TTC 1329
Met Ser Lys Glu Lys Ala Arg Tyr Phe
1 5

ACC TTT TTG CTT TAT CCA GAA AGC ATA CCA AAT GAC TGG ATA GAT AAC 1377
Thr Phe Leu Leu Tyr Pro Glu Ser Ile Pro Asn Asp Trp Ile Asp Asn
10 15 20 25

TTA GAA TTA ATA GGT GTT CCG ATT GCT ATT AGT CCT CTG CAT GAT AAG 1425
Leu Glu Leu Ile Gly Val Pro Ile Ala Ile Ser Pro Leu His Asp Lys
30 35 40

GAT TTA AGT GAT GTT GAG GGA CAA AAA TAT AAA AAA GAA CAT TAT CAT 1473
Asp Leu Ser Asp Val Glu Gly Gln Lys Tyr Lys Lys Glu His Tyr His
45 50 55

GTA ATC TAT GTT TCA AAA AAT CCT GTA ACT GCT GAA AGT GTG CGT TTG 1521
Val Ile Tyr Val Ser Lys Asn Pro Val Thr Ala Glu Ser Val Arg Leu
60 65 70

AAA GTT AAG CGC TCT TTG GGA GAT AAA AGT GTT GCT ATG GTT CAG ATT 1569
Lys Val Lys Arg Ser Leu Gly Asp Lys Ser Val Ala Met Val Gln Ile
75 80 85

GTA AGT ACG AGT ATG GAA AAT ATG TAT TTG TAT TTA ACG CAT GAA TCA 1617
Val Ser Thr Ser Met Glu Asn Met Tyr Leu Tyr Leu Thr His Glu Ser
90 95 100 105

AAA GAC GCT ATT GCT AAA AAT AAG CAT AAA TAT TCT AAA GCT GAT ATT 1665
Lys Asp Ala Ile Ala Lys Asn Lys His Lys Tyr Ser Lys Ala Asp Ile
110 115 120

AGG TTG TTG AAT AAT TTT GAT ATT GAT CGT TAT GTA ACG CTT GAC GTC 1713
Arg Leu Leu Asn Asn Phe Asp Ile Asp Arg Tyr Val Thr Leu Asp Val
125 130 135

GAA GAT AAA GAC GAT ATG TTG AAT GAT GTT TGC GAT TTA ATT GAT GAT 1761
Glu Asp Lys Asp Asp Met Leu Asn Asp Val Cys Asp Leu Ile Asp Asp
140 145 150

CAT AAT TTA GCA AAT ATG CGT GAA TTA AGG CGC TTT GTT AAA TTA CAT 1809
His Asn Leu Ala Asn Met Arg Glu Leu Arg Arg Phe Val Lys Leu His
155 160 165

GGG GTT GAA TAT GGC TTG CCT AGT ATG AAA ATT ATA AAT TCA GTG TTG 1857
Gly Val Glu Tyr Gly Leu Pro Ser Met Lys Ile Ile Asn Ser Val Leu
170 175 180 185

AGA GCT CAT ACT GGA CTG ATA AGG TTA TAT TTC GAT GCT GTT TAT CAA 1905
Arg Ala His Thr Gly Leu Ile Arg Leu Tyr Phe Asp Ala Val Tyr Gln
190 195 200

GAA CGT CGA TAT GGT CGG GCA GAT ATT GAT AAA GAT ACA GGC GAA GTA 1953
Glu Arg Arg Tyr Gly Arg Ala Asp Ile Asp Lys Asp Thr Gly Glu Val
205 210 215

TTG AAT AAT AAG TAAGGGTTTA ATTTTATTTT AGGGGGAAGC AATT ATG AAA 2005
Leu Asn Asn Lys Met Lys
220 1

TTT GAA GAA ACA TTA TTA CCA GAA AAA TCA GAC GTA ATG ACA TTA CAA 2053
Phe Glu Glu Thr Leu Leu Pro Glu Lys Ser Asp Val Met Thr Leu Gln
5 10 15

AAT ATG ATC AGA AAG TAT AAT AAG CAA AAT TTT GAA ACT GCA AAT CAA 2101
Asn Met Ile Arg Lys Tyr Asn Lys Gln Asn Phe Glu Thr Ala Asn Gln
20 25 30

ACA GAT TTT GCT ATT TAC ATA AAA GAT GAT AGT GAG AAT GTT ATG GGG 2149
Thr Asp Phe Ala Ile Tyr Ile Lys Asp Asp Ser Glu Asn Val Met Gly
35 40 45 50

GGG ATT TCA GGA AAA ATA TTT GGT AAT TGG ATG GAT ATA GAC TAT TTA 2197
Gly Ile Ser Gly Lys Ile Phe Gly Asn Trp Met Asp Ile Asp Tyr Leu
55 60 65

GTT ATT CAT GAG AGT TTG CGC AAA AAT GGG TTA GGT CGG GAT TTG TTA 2245
Val Ile His Glu Ser Leu Arg Lys Asn Gly Leu Gly Arg Asp Leu Leu
70 75 80

AAA AAA GCC GAA GAT TTA GCT ATA CGG AGT AAT TGT AAA AAT ATA TTT 2293
Lys Lys Ala Glu Asp Leu Ala Ile Arg Ser Asn Cys Lys Asn Ile Phe
85 90 95

TTA TAT ACA TTT GAT TTT CAA GGT AAA GAT TTT TAT CCC AAA TTT GGA 2341
Leu Tyr Thr Phe Asp Phe Gln Gly Lys Asp Phe Tyr Pro Lys Phe Gly
100 105 110

TTT AAA GAA GTT TAC GTA AAA AGA CAG TAT CCG TTA ACT GGA ACA GAA 2389
Phe Lys Glu Val Tyr Val Lys Arg Gln Tyr Pro Leu Thr Gly Thr Glu
115 120 125 130

CAT TTT TTT GTT AAG AAA TTA TCA AAT GAA ATC GAT AAA GAA ACT GAA 2437
His Phe Phe Val Lys Lys Leu Ser Asn Glu Ile Asp Lys Glu Thr Glu
135 140 145

TGATGTGATG AAAGGACGGA TAACTAGATC CGTCCTTTTT TGTTGTCGAT TA 2489

(2) INFORMATION FOR SEQ ID NO: 2:

(i) SEQUENCE CHARACTERISTICS:
(A) LENGTH: 56 amino acids
(B) TYPE: amino acid
(D) TOPOLOGY: linear

(ii) MOLECULE TYPE: protein

(xi) SEQUENCE DESCRIPTION: SEQ ID NO: 2:

Met Ala Arg Glu Lys Ser Thr Ile Glu Tyr Gln Asn Val Ser Met Arg
1               5                   10                  15

Ile Pro Lys Glu Leu Tyr Ser Glu Tyr Lys Lys Val Leu Ala Lys Thr
            20                  25                  30

Gly Ser Ile Val Thr Tyr Asp Val Arg Asn Tyr Met Lys Lys Val Val
        35                  40                  45

Glu Glu Asp Lys Lys Gly Gln Lys
    50                  55

(2) INFORMATION FOR SEQ ID NO: 3:

(i) SEQUENCE CHARACTERISTICS:
(A) LENGTH: 221 amino acids
(B) TYPE: amino acid
(D) TOPOLOGY: linear

(ii) MOLECULE TYPE: protein

(xi) SEQUENCE DESCRIPTION: SEQ ID NO: 3:

Met Ser Lys Glu Lys Ala Arg Tyr Phe Thr Phe Leu Leu Tyr Pro Glu
1               5                   10                  15

Ser Ile Pro Asn Asp Trp Ile Asp Asn Leu Glu Leu Ile Gly Val Pro
            20                  25                  30

Ile Ala Ile Ser Pro Leu His Asp Lys Asp Leu Ser Asp Val Glu Gly
        35                  40                  45

Gln Lys Tyr Lys Lys Glu His Tyr His Val Ile Tyr Val Ser Lys Asn
    50                  55                  60

Pro Val Thr Ala Glu Ser Val Arg Leu Lys Val Lys Arg Ser Leu Gly
65                  70                  75                  80

Asp Lys Ser Val Ala Met Val Gln Ile Val Ser Thr Ser Met Glu Asn
                85                  90                  95

Met Tyr Leu Tyr Leu Thr His Glu Ser Lys Asp Ala Ile Ala Lys Asn
            100                 105                 110

Lys His Lys Tyr Ser Lys Ala Asp Ile Arg Leu Leu Asn Asn Phe Asp
        115                 120                 125

Ile Asp Arg Tyr Val Thr Leu Asp Val Glu Asp Lys Asp Asp Met Leu
    130                 135                 140

Asn Asp Val Cys Asp Leu Ile Asp Asp His Asn Leu Ala Asn Met Arg
145                 150                 155                 160

Glu Leu Arg Arg Phe Val Lys Leu His Gly Val Glu Tyr Gly Leu Pro
                165                 170                 175

Ser Met Lys Ile Ile Asn Ser Val Leu Arg Ala His Thr Gly Leu Ile
            180                 185                 190

Arg Leu Tyr Phe Asp Ala Val Tyr Gln Glu Arg Arg Tyr Gly Arg Ala
        195                 200                 205

Asp Ile Asp Lys Asp Thr Gly Glu Val Leu Asn Asn Lys
    210                 215                 220

(2) INFORMATION FOR SEQ ID NO: 4:

(i) SEQUENCE CHARACTERISTICS:
(A) LENGTH: 146 amino acids
(B) TYPE: amino acid
(D) TOPOLOGY: linear

(ii) MOLECULE TYPE: protein
(xi) SEQUENCE DESCRIPTION: SEQ ID NO: 4:

Met Lys Phe Glu Glu Thr Leu Leu Pro Glu Lys Ser Asp Val Met Thr
1               5                   10                  15

Leu Gln Asn Met Ile Arg Lys Tyr Asn Lys Gln Asn Phe Glu Thr Ala
            20                  25                  30

Asn Gln Thr Asp Phe Ala Ile Tyr Ile Lys Asp Asp Ser Glu Asn Val
        35                  40                  45

Met Gly Gly Ile Ser Gly Lys Ile Phe Gly Asn Trp Met Asp Ile Asp
    50                  55                  60

Tyr Leu Val Ile His Glu Ser Leu Arg Lys Asn Gly Leu Gly Arg Asp
65                  70                  75                  80

Leu Leu Lys Lys Ala Glu Asp Leu Ala Ile Arg Ser Asn Cys Lys Asn
                85                  90                  95

Ile Phe Leu Tyr Thr Phe Asp Phe Gln Gly Lys Asp Phe Tyr Pro Lys
            100                 105                 110

Phe Gly Phe Lys Glu Val Tyr Val Lys Arg Gln Tyr Pro Leu Thr Gly
        115                 120                 125

Thr Glu His Phe Phe Val Lys Lys Leu Ser Asn Glu Ile Asp Lys Glu
    130                 135                 140

Thr Glu
145

(2) INFORMATION FOR SEQ ID NO: 5:

(i) SEQUENCE CHARACTERISTICS:
(A) LENGTH: 3213 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO: 5:

CATGGTTTTA TTCGTGCGGG ATAGAAATTT GGAAATCAGG GGGGCGAGGG AGCGAATTTT 60
GCGACCGTAC TACGACCCCC CTTTTAAGTG CCGAGTGCCA AATCGGTCAA CAAACCCTGA 120

CAAACCCATA CACACCAAGT GTTACAGCGA TCAACAAAAA GTCAACAACC CGCCAACAAA 180

ATTTTGGAAA TATAATGATA AAAAGGTAGT TGAATATATA TATATAAAGT TGTATAATGT 240

TCTTATGAAC ATAAAAGGAG TGTTATAAAT GGCTAGAGAG AAATCAACGA TAGAATACCA 300

AAATGTATCA ATGCGTATTC CCAAAGAATT ATATTCTGAA TATAAAAAAG TTTTGGCTAA 360

AACTGGATCT ATTGTTACTT ATGACGTTCG AAATTATATG AAAAAAGTTG TTGAAGAAGA 420

TAAGAAAGGA CAAAAATGAG TATAAAAAAA GAGCGCCCTC TTCGAAAAGT CGCTCATGTT 480

TTTATAATGT TTTTACTAAT TTTTGTAAGG TAATTATATC AAATGAGTAA AGAAAAAGCT 540

AGATACTTCA CCTTTTTGCT TTATCCAGAA AGCATACCAA ATGACTGGAT AGATAACTTA 600

GAATTAATAG GTGTTCCGAT TGCTATTAGT CCTCTGCATG ATAAGGATTT AAGTGATGTT 660

GAGGGACAAA AATATAAAAA AGAACATTAT CATGTAATCT ATGTTTCAAA AAATCCTGTA 720

ACTGCTGAAA GTGTGCGTTT GAAAGTTAAG CGCTCTTTGG GAGATAAAAG TGTTGCTATG 780

GTTCAGATTG TAAGTACGAG TATGGAAAAT ATGTATTTGT ATTTAACGCA TGAATCAAAA 840

GACGCTATTG CTAAAAATAA GCATAAATAT TCTAAAGCTG ATATTAGGTT GTTGAATAAT 900

TTTGATATTG ATCGTTATGT AACGCTTGAC GTCGAAGATA AAGACGATAT GTTGAATGAT 960

GTTTGCGATT TAATTGATGA TCATAATTTA GCAAATATGC GTGAATTAAG GCGCTTTGTT 1020

AAATTACATG GGGTTGAATA TGGCTTGCCT AGTATGAAAA TTATAAATTC AGTGTTGAGA 1080

GCTCATACTG GACTGATAAG GTTATATTTC GATGCTGTTT ATCAAGAACG TCGATATGGT 1140

CGGGCAGATA TTGATAAAGA TACAGGCGAA GTATTGAATA ATAAGTAAGG GTTTAATTTT 1200

ATTTTAGGGG GAAGCAATTA TGAAATTTGA AGAAACATTA TTACCAGAAA AATCAGACGT 1260

AATGACATTA CAAAATATGA TCAGAAAGTA TAATAAGCAA AATTTTGAAA CTGCAAATCA 1320

AACAGATTTT GCTATTTACA TAAAAGATGA TAGTGAGAAT GTTATGGGGG GGATTTCAGG 1380

AAAAATATTT GGTAATTGGA TGGATATAGA CTATTTAGTT ATTCATGAGA GTTTGCGCAA 1440

AAATGGGTTA GGTCGGGATT TGTTAAAAAA AGCCGAAGAT TTAGCTATAC GGAGTAATTG 1500

TAAAAATATA TTTTTATATA CATTTGATTT TCAAGGTAAA GATTTTTATC CCAAATTTGG 1560

ATTTAAAGAA GTTTACGTAA AAAGACAGTA TCCGTTAACT GGAACAGAAC ATTTTTTTGT 1620

TAAGAAATTA TCAAATGAAA TCGGATCGAC GGATCCGGGG AATTCACTGG AACATTCAAA 1680

CGGCCTGTCT CATCATGGAA GGCGCTGAAT TTACGGAAAA CATTATTAAT GGCGTCGAGC 1740

GTCCGGTTAA AGCCGCTGAA TTGTTCGCGT TTACCTTGCG TGTACGCGCA GGAAACACTG 1800

ACGTTCTTAC TGACGCAGAA GAAAACGTGC GTCAAAAATT ACGTGCGGAA GGAGTGATGT 1860

AATGTCTAAA GGTAAAAAAC GTTCTGGCGC TCGCCCTAAT TCACTGGCCG TCGTTTTACA 1920

ACGTCGTGAC TGGGAAAACC CTGGCGTTAC CCAACTTAAT CGCCTTGCAG CACATCCCCC 1980

TTTCGCCAGC TGGCGTAATA GCGAAGAGGC CCGCACCGAT CGCCCTTCCC AACAGTTGCG 2040

CAGCCTGAAT GGCGAATGGC GCCTGATGCG GTATTTTCTC CTTACGCATC TGTGCGGTAT 2100

TTCACACCGC ATACGGCAAT AGTTACCCTT ATTATCAAGA TAAGAAAGAA AAGGATTTTT 2160

CGCTACGCTC AAATCCTTTA AAAAAACACA AAAGACCACA TTTTTTAATG TGGTCTTTAT 2220

TCTTCAACTA AAGCACCCAT TAGTTCAACA AACGAAAATT GGATAAAGTG GGATATTTTT 2280

AAAATATATA TTTATGTTAC AGTAATATTG ACTTTTAAAA AAGGATTGAT TCTAATGAAG 2340

AAAGCAGACA AGTAAGCCTC CTAAATTCAC TTTAGATAAA AATTTAGGAG GCATATCAAA 2400

TGAACTTTAA TAAAATTGAT TTAGACAATT GGAAGAGAAA AGAGATATTT AATCATTATT 2460

TGAACCAACA AACGACTTTT AGTATAACCA CAGAAATTGA TATTAGTGTT TTATACCGAA 2520

ACATAAAACA AGAAGGATAT AAATTTTACC CTGCATTTAT TTTCTTAGTG ACAAGGGTGA 2580

TAAACTCAAA TACAGCTTTT AGAACTGGTT ACAATAGCGA CGGAGAGTTA GGTTATTGGG 2640

ATAAGTTAGA GCCACTTTAT ACAATTTTTG ATGGTGTATC TAAAACATTC TCTGGTATTT 2700

GGACTCCTGT AAAGAATGAC TTCAAAGAGT TTTATGATTT ATACCTTTCT GATGTAGAGA 2760

AATATAATGG TTCGGGGAAA TTGTTTCCCA AAACACCTAT ACCTGAAAAT GCTTTTTCTC 2820

TTTCTATTAT TCCATGGACT TCATTTACTG GGTTTAACTT AAATATCAAT AATAATAGTA 2880

ATTACCTTCT ACCCATTATT ACAGCAGGAA AATTCATTAA TAAAGGTAAT TCAATATATT 2940

TACCGCTATC TTTACAGGTA CATCATTCTG TTTGTGATGG TTATCATGCA GGATTGTTTA 3000

TGAACTCTAT TCAGGAATTG TCAGATAGGC CTAATGACTG GCTTTTATAA TATGAGATAA 3060

TGCCGACTGT ACTTTTTACA GTCGGTTTTC TAATGTCACT AACCTGCCCC GTTAGTTGAA 3120

GAAGGTTTTT ATATTACAGC TCCAGATCTC GAGGGTTACC ATCGATCGGC CGAAGCTTGG 3180

TACCATGCAT GCGTCGACTC TAGACTGCAG GTC 3213

(2) INFORMATION FOR SEQ ID NO: 6:

(i) SEQUENCE CHARACTERISTICS:
(A) LENGTH: 3213 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO: 6:

CATGGTTTTA TTCGTGCGGG ATAGAAATTT GGAAATCAGG GGGGCGAGGG AGCGAATTTT 60

GCGACCGTAC TACGACCCCC CTTTTAAGTG CCGAGTGCCA AATCGGTCAA CAAACCCTGA 120

CAAACCCATA CACACCAAGT GTTACAGCGA TCAACAAAAA GTCAACAACC CGCCAACAAA 180

ATTTTGGAAA TATAATGATA AAAAGGTAGT TGAATATATA TATATAAAGT TGTATAATGT 240

TCTTATGAAC ATAAAAGGAG TGTTATAAAT GGCTAGAGAG AAATCAACGA TAGAATACCA 300

AAATGTATCA ATGCGTATTC CCAAAGAATT ATATTCTGAA TATAAAAAAG TTTTGGCTAA 360

AACTGGATCT ATTGTTACTT ATGACGTTCG AAATTATATG AAAAAAGTTG TTGAAGAAGA 420

TAAGAAAGGA CAAAAATGAG TATAAAAAAA GAGCGCCCTC TTCGAAAAGT CGCTCATGTT 480

TTTATAATGT TTTTACTAAT TTTTGTAAGG TAATTATATC AAATGAGTAA AGAAAAAGCT 540

AGATACTTCA CCTTTTTGCT TTATCCAGAA AGCATACCAA ATGACTGGAT AGATAACTTA 600

GAATTAATAG GTGTTCCGAT TGCTATTAGT CCTCTGCATG ATAAGGATTT AAGTGATGTT 660

GAGGGACAAA AATATAAAAA AGAACATTAT CATGTAATCT ATGTTTCAAA AAATCCTGTA 720

ACTGCTGAAA GTGTGCGTTT GAAAGTTAAG CGCTCTTTGG GAGATAAAAG TGTTGCTATG 780

GTTCAGATTG TAAGTACGAG TATGGAAAAT ATGTATTTGT ATTTAACGCA TGAATCAAAA 840

GACGCTATTG CTAAAAATAA GCATAAATAT TCTAAAGCTG ATATTAGGTT GTTGAATAAT 900

TTTGATATTG ATCGTTATGT AACGCTTGAC GTCGAAGATA AAGACGATAT GTTGAATGAT 960

GTTTGCGATT TAATTGATGA TCATAATTTA GCAAATATGC GTGAATTAAG GCGCTTTGTT 1020

AAATTACATG GGGTTGAATA TGGCTTGCCT AGTATGAAAA TTATAAATTC AGTGTTGAGA 1080

GCTCATACTG GACTGATAAG GTTATATTTC GATGCTGTTT ATCAAGAACG TCGATATGGT 1140

CGGGCAGATA TTGATAAAGA TACAGGCGAA GTATTGAATA ATAAGTAAGG GTTTAATTTT 1200

ATTTTAGGGG GAAGCAATTA TGAAATTTGA AGAAACATTA TTACCAGAAA AATCAGACGT 1260

AATGACATTA CAAAATATGA TCAGAAAGTA TAATAAGCAA AATTTTGAAA CTGCAAATCA 1320

AACAGATTTT GCTATTTACA TAAAAGATGA TAGTGAGAAT GTTATGGGGG GGATTTCAGG 1380

AAAAATATTT GGTAATTGGA TGGATATAGA CTATTTAGTT ATTCATGAGA GTTTGCGCAA 1440

AAATGGGTTA GGTCGGGATT TGTTAAAAAA AGCCGAAGAT TTAGCTATAC GGAGTAATTG 1500

TAAAAATATA TTTTTATATA CATTTGATTT TCAAGGTAAA GATTTTTATC CCAAATTTGG 1560

ATTTAAAGAA GTTTACGTAA AAAGACAGTA TCCGTTAACT GGAACAGAAC ATTTTTTTGT 1620

TAAGAAATTA TCAAATGAAA TCGGATCGAC GGATCCGGGG AATTCACTGG AACATTCAAA 1680

CGGCCTGTCT CATCATGGAA GGCGCTGAAT TTACGGAAAA CATTATTAAT GGCGTCGAGC 1740

GTCCGGTTAA AGCCGCTGAA TTGTTCGCGT TTACCTTGCG TGTACGCGCA GGAAACACTG 1800

ACGTTCTTAC TGACGCAGAA GAAAACGTGC GTCAAAAATT ACGTGCGGAA GGAGTGATGT 1860

AATGTCTAAA GGTAAAAAAC GTTCTGGCGC TCGCCCTAAT TCACTGGCCG TCGTTTTACA 1920

ACGTCGTGAC TGGGAAAACC CTGGCGTTAC CCAACTTAAT CGCCTTGCAG CACATCCCCC 1980

TTTCGCCAGC TGGCGTAATA GCGAAGAGGC CCGCACCGAT CGCCCTTCCC AACAGTTGCG 2040

CAGCCTGAAT GGCGAATGGC GCCTGATGCG GTATTTTCTC CTTACGCATC TGTGCGGTAT 2100

TTCACACCGC ATACGGCAAT AGTTACCCTT ATTATCAAGA TAAGAAAGAA AAGGATTTTT 2160

CGCTACGCTC AAATCCTTTA AAAAAACACA AAAGACCACA TTTTTTAATG TGGTCTTTAT 2220

TCTTCAACTA AAGCACCCAT TAGTTCAACA AACGAAAATT GGATAAAGTG GGATATTTTT 2280

AAAATATATA TTTATGTTAC AGTAATATTG ACTTTTAAAA AAGGATTGAT TCTAATGAAG 2340

AAAGCAGACA AGTAAGCCTC CTAAATTCAC TTTAGATAAA AATTTAGGAG GCATATCAAA 2400

TGAACTTTAA TAAAATTGAT TTAGACAATT GGAAGAGAAA AGAGATATTT AATCATTATT 2460

TGAACCAACA AACGACTTTT AGTATAACCA CAGAAATTGA TATTAGTGTT TTATACCGAA 2520

ACATAAAACA AGAAGGATAT AAATTTTACC CTGCATTTAT TTTCTTAGTG ACAAGGGTGA 2580

TAAACTCAAA TACAGCTTTT AGAACTGGTT ACAATAGCGA CGGAGAGTTA GGTTATTGGG 2640

ATAAGTTAGA GCCACTTTAT ACAATTTTTG ATGGTGTATC TAAAACATTC TCTGGTATTT 2700

GGACTCCTGT AAAGAATGAC TTCAAAGAGT TTTATGATTT ATACCTTTCT GATGTAGAGA 2760

AATATAATGG TTCGGGGAAA TTGTTTCCCA AAACACCTAT ACCTGAAAAT GCTTTTTCTC 2820

TTTCTATTAT TCCATGGACT TCATTTACTG GGTTTAACTT AAATATCAAT AATAATAGTA 2880

ATTACCTTCT ACCCATTATT ACAGCAGGAA AATTCATTAA TAAAGGTAAT TCAATATATT 2940

TACCGCTATC TTTACAGGTA CATCATTCTG TTTGTGATGG TTATCATGCA GGATTGTTTA 3000

TGAACTCTAT TCAGGAATTG TCAGATAGGC CTAATGACTG GCTTTTATAA TATGAGATAA 3060

TGCCGACTGT ACTTTTTACA GTCGGTTTTC TAATGTCACT AACCTGCCCC GTTAGTTGAA 3120

GAAGGTTTTT ATATTACAGC TCCAGATCCT AGAGTCGACG CATGCATGGT ACCAAGCTTC 3180

GGCCGATCGA TGGTAACCCT CGAGCTGCAG GTC 3213

## Description

The invention relates to vectors for the transformation of cellular hosts such as lactobacilli, said vectors comprising all or part of the nucleotide sequence of a plasmid isolated from *Lactobacillus curvatus*.

The invention also relates to processes for the production of predetermined polypeptides by transformation of appropriate cellular hosts with said vectors. The invention relates more particularly to the use of said vectors in fermented foodstuff preparation processes.

The invention also relates to proteins coded by the genome of said vectors.

In recent years there has been an upsurge in interest into the possibility of genetically manipulating lactic acid bacteria (McKay et al., 1990). Many species are now transformable by using the electroporation technique and thus become amenable to recombinant DNA technology. A strategy for the construction of cloning vectors, is to use the origins of replication of small multicopy cryptic plasmids from lactobacilli and to incorporate selectable markers.

Very little is known about the replication mode of plasmids in lactobacilli. Characterizations of plasmids isolated from different *Lactobacillus* species (Bates and Gilbert, 1989; Cocconelli et al. 1991; Josson et al., 1990; Leer et al, 1992; Skaugen, 1989) have revealed that they may belong to the group of single-stranded (ss) DNA plasmids that replicate via a RCR mechanism (Gruss and Ehrlich, 1989; Te Riele et al., 1986). The nucleotide sequence data indicate that all *Lactobacillus* plasmids analysed so far have a similar organization as ssDNA plasmids from other Gram positive bacteria. All plasmids encode a site specific nuclease, the Rep protein, which produces a specific single stranded nick at the plus origin, that allows initiation of synthesis of the leading DNA strand. Depending on the similarity of plus origin sequences, the various plasmids have been classified into three groups (Gruss and Ehrlich, 1989). Several *Lactobacillus* plasmids contain palindromic structures which resemble the minus origin (MO) of replication in ssDNA plasmids. The MOs are involved in the conversion of ssDNA into double-stranded (ds) DNA. These nonessential regions have been distinguished into three classes (Gruss and Ehrlich, 1989) but additionally MOs of *Lactobacillus* plasmids have been described, which have no homology to MOs of other ssDNA plasmids (Leer et al., 1992). Since inserts of foreign DNA might influence plasmid stability (Gruss and Ehrlich, 1988) efforts were made to isolate large lactobacillal plasmids replicating via a double stranded theta-type mechanism (Janniére et al., 1990) as it is described for the well characterized plasmid pAMB1 isolated from *Enterococcus faecalis* (Leblanc and Lee, 1984), but until now no report of such a plasmid has been published.

*Lactobacillus curvatus* is a commercially important microorganism in sausage fermentation and like many other lactobacilli this species harbours cryptic plasmids. The presence of plasmid DNA in *Lb. curvatus* strains has been reported (Vogel et al., 1991) and plasmid transfer and segregation of transformed vector plasmids was investigated (Vogel et al., 1992). The segregationally highly stable small cryptic plasmid pLC2 of *Lb. curvatus* LTH683 (formerly LC2) could serve as a minimal replicon in food-grade vectors for use in lactobacilli, especially for *Lactobacillus curvatus,* a species with a plasmid cured and transformable strain (Gaier et al., 1990). Recently a catalase gene of this species was cloned, which has potential to be a food grade marker gene (Knauf et al., 1992), but until now no vector with a replicon originating from this species was available.

An aspect of the invention is to provide a new family of nucleic acids, which nucleic acids when constituting or being part of the genome of a vector, allows this latter to replicate in a cellular host.

Another aspect of the invention is to provide vectors the genome of which comprises said nucleic acids.

Another aspect of the invention is to provide cellular hosts transformed with said vectors, and which can be used for the preparation of predetermined polypeptides, more particularly in fermented foodstuff preparation processes.

Another aspect of the invention is to provide new proteins coded by the genome of said vectors, said proteins being useful for the replication of these vectors.

Another aspect of the invention is to provide processes for the preparation of predetermined polypeptides.

Another aspect of the invention is to provide fermentation processes, such as processes for the preparation of fermented foodstuff, and more particularly cheese.

The invention will be illustrated by the following figures:
- figure 1 represents the nucleotide sequence of plasmid pLC2, together with the amino acid sequences encoded by pLC2, these amino acid sequences being represented under the nucleotide sequence,
- figure 2 represents the schematic alignement of pLC2 nucleotide sequence with homologous regions of plasmids from Gram bacteria,
- figure 3 represents the comparison of plasmid plus *ori* sites,
- figure 4 represents the determination of the minimal replicon of pLC2,
- figure 5 represents an autoradiogramm of labelled proteins of the invention,
- figure 6 represents cell lysates of *Lb. curvatus* LTH683,
- figures 7 and 8 represent the construction of cloning vectors pJK355 and pJK356,
- figure 9 represents the replacement of the chloramphenicol resistance gene in pJK355 with a selectable food grade marker.

The invention relates to nucleic acids characterized in that they comprise:
- all or part of the nucleotide sequence represented by SEQ ID NO: 1, or its complementary sequence,
- or a nucleotide sequence susceptible to hybridize with all or part of the preceeding nucleotide sequences, more particularly a nucleotide sequence which differs from the preceeding ones by addition and/or suppression and/or substitution of one or several nucleotides,
said nucleic acids, when constituting the genome of a vector, allow this latter to replicate in a cellular host.

The invention relates more particularly to nucleic acids such as described above and characterized in that they comprise the nucleotide sequence represented by SEQ ID NO: 1, or fragments of said nucleotide sequence, these fragments, alone or in combination with one or several other fragments of said nucleotide sequence, being still able to allow the replication of said vector.

Preferred nucleic acids according to the invention are characterized in that they comprise all or part of the nucleotide sequence delimited by the nucleotide located at the position 780 and the nucleotide located at the position 1909 of the nucleotide sequence represented by SEQ ID NO: 1.

The invention relates more particularly to nucleic acids characterized in that they comprise all or part of the nucleotide sequence delimited by the nucleotide located at the position 813 and the nucleotide located at the position 869 of the nucleotide sequence represented by SEQ ID NO: 1, and more particularly the TACTACGA sequence located at positions 848-855, corresponding to a potential plus origin of replication.

The invention relates more particularly to nucleic acids characterized in that they comprise all or part of the nucleotide sequence delimited by the nucleotide located at the position 305 and the nucleotide located at the position 640 of the nucleotide sequence represented by SEQ ID NO: 1, and more particularly the sequence located at positions 574-640, corresponding to a potential minus origin of replication.

The invention relates more particularly to nucleic acids, characterized in that they comprise all or part of the nucleotide sequence delimited by the nucleotide located at the position 1049 and the nucleotide located at the position 1216 of the nucleotide sequence represented by SEQ ID NO 1, and coding for a protein of 56 amino acids represented by SEQ ID NO: 2.

The invention relates more particularly to nucleic acids comprising all or part of the nucleotide sequence delimited by the nucleotide located at the position 1049 and the nucleotide located at the position 1216 of the nucleotide sequence represented by SEQ ID NO: 1, such as defined above, and characterized in that they comprise also all or part of the nucleotide sequence delimited by the nucleotide located at the position 1027 and the nucleotide located at the position 1048 of the nucleotide sequence represented by SEQ ID NO: 1, and more particularly the sequence AGGGA at positions 1036-1040, corresponding to a potential ribosome binding site.

The invention relates more particularly to nucleic acids comprising all or part of the nucleotide sequence delimited by the nucleotide located at the position 1049 and the nucleotide located at the position 1216, and all or part of the nucleotide sequence delimited by the nucleotide located at the position 1049 and the nucleotide located at the position 1216 of the nucleotide sequence represented by SEQ ID NO: 1, such as defined above, and characterized in that they comprise also all or part of the nucleotide sequence delimited by the nucleotide located at the position 958 and the nucleotide located at the position 1026 of the nucleotide sequence represented by SEQ ID NO: 1, and corresponding to a promoter.

The invention relates more particularly to nucleic acids, characterized in that they comprise all or part of the nucleotide sequence delimited by the nucleotide located at the position 1303 and the nucleotide located at the position 1965 of the nucleotide sequence represented by SEQ ID NO: 1, and coding for a protein of 221 amino acids represented by SEQ ID NO: 3.

The invention relates more particularly to nucleic acids comprising all or part of the nucleotide sequence delimited by the nucleotide located at the position 1303 and the nucleotide located at the position 1965 of the nucleotide sequence represented by SEQ ID NO: 1, such as defined above, and characterized in that they comprise also all or part of the nucleotide sequence delimited by the nucleotide located at the position 1286 and the nucleotide located at the position 1302 of the nucleotide sequence represented by SEQ ID NO: 1, and more particularly the sequence TAAGG at positions 1286-1290, corresponding to a potential ribosome binding site.

The invention relates more particularly to nucleic acids comprising all or part of the nucleotide sequence delimited by the nucleotide located at the position 1303 and the nucleotide located at the position 1965, and all or part of the nucleotide sequence delimited by the nucleotide located at the position 1286 and the nucleotide located at the position 1302 of the nucleotide sequence represented by SEQ ID NO: 1, such as defined above, and characterized in that they comprise also all or part of the nucleotide sequence delimited by the nucleotide located at the position 1217 and the nucleotide located at the position 1285 of the nucleotide sequence represented by SEQ ID NO: 1, and more partcularly the sequences at positions 1219-1232 and positions 1263-1269, corresponding to a promoter.

The invention relates more particularly to nucleic acids characterized in that they comprise all or part of the nucleotide sequence delimited by the nucleotide located at the position 2000 and the nucleotide located at the position 2437 of the nucleotide sequence represented by SEQ ID NO: 1, and coding for a protein of 146 amino acids represented by SEQ ID NO: 4.

The invention relates more particularly to nucleic acids such as defined above, and characterized in that they comprise all or part of the nucleotide sequence delimited by the nucleotide located at the position 2448 and the nucleotide located at the position 2475 of the nucleotide sequence represented SEQ ID NO: 1, corresponding to a potential translational promoter.

The invention relates more particularly to nucleic acids characterized in that they comprise all or part of the nucleotide sequence delimited by the nucleotide located at the position 781 and the nucleotide located at the position 2423 of the nucleotide sequence represented by SEQ ID NO: 1.

The invention also relates to replicons constituted by nucleic acids of the invention, and more particularly to the replicon constituted by the nucleotide sequence delimited by the nucleotide located at the position 781 and the nucleotide located at the position 2423 of the nucleotide sequence represented by SEQ ID NO: 1.

The invention also relates to the protein comprising the 56 amino acids sequence represented by SEQ ID NO: 2, and coded by the nucleotide sequence described above and delimited by the nucleotide located at the position 1049 and the nucleotide located at the position 1216 of the nucleotide sequence represented by SEQ ID NO: 1, this protein being able to allow the replication of a vector, the genome of which codes for such a protein, in a cellular host, or fragments or muteins (which differ from said protein by addition and/or substitution and/or suppression of one or several amino acids) thereof, provided that said fragments or muteins are still able to allow the replication of said vector.

The invention also relates to the protein comprising the 221 amino acids sequence represented by SEQ ID NO 3, and coded by the nucleotide sequence described above and delimited by the nucleotide located at the position 1303 and the nucleotide located at the position 1965 of the nucleotide sequence represented by SEQ ID NO: 1, this protein being able to allow the replication of a vector, the genome of which codes for such a protein, in a cellular host, or fragments or muteins thereof such as defined above.

The invention also relates to the protein comprising the 146 amino acids sequence represented by SEQ ID NO; 4, and coded by the nucleotide sequence defined above and delimited by the nucleotide located at the position 2000 and the nucleotide located at the position 2437 of the nucleotide sequence represented by SEQ ID NO: 1, this protein being able to allow the replication of a vector, the genome of which codes for such a protein, in a cellular host, or fragments or muteins thereof such as defined above.

The invention relates to nucleotide sequences coding for all or part of the proteins represented by SEQ ID NO: 2, SEQ ID NO: 3 and SEQ ID NO: 4 cited above, and more particularly to nucleotide sequences, defined above, coding for said proteins, or to nucleotide sequences differing from those latter according to the degeneracy of the genetic code.

From the nucleic acids of the invention, probes (i.e. cloned or synthetic oligonucleotides) can be inferred.

These probes can be from 25 nucleotides up to the total length of the nucleic acids described above.

These oligonucleotides can also be used as amplification primers in the PCR technique (PCR, Mullis and Faloma, Methods in Enzymology, vol. 155, p. 335, 1987) and generate specific enzymatially amplified fragments and/or as probes to detect fragments amplified between bracketing oligonucleotide primers.

The invention also relates to vectors, and more particularly to plasmids, susceptible to replicate in a cellular host, characterized in that their genome comprises a nucleic acid according to the invention.

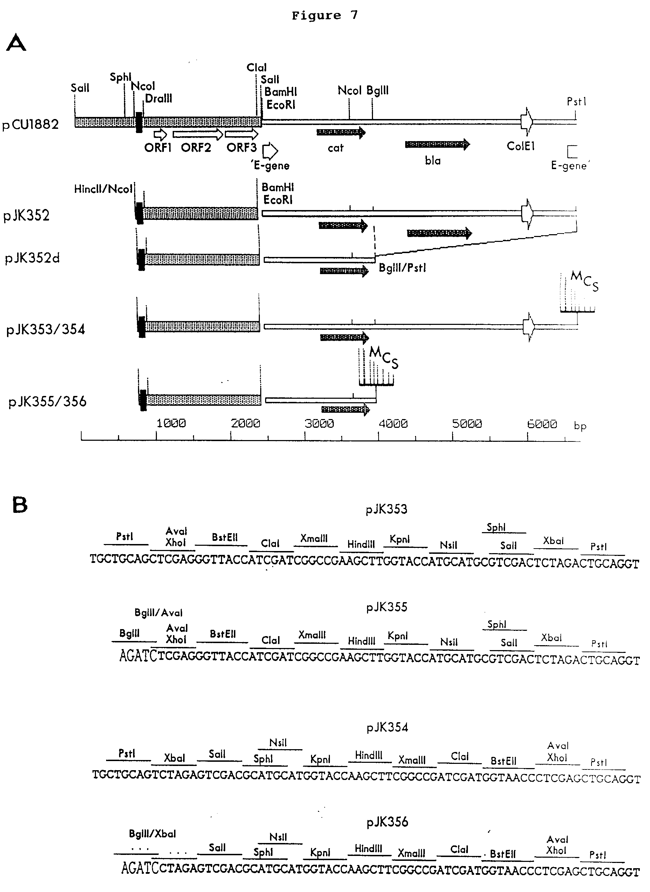

Preferred vectors according to the invention are characterized in that this genome comprises one (or several) multiple cloning site(s) such as those represented on figure 7B.

The invention relates more particularly to plasmids pJK355 and pJK356 used for the transformation of the strain LK1 of *Lactobacillus casei*, these strains thus transformed being deposited at the Deutsche Sammlung von Mikroorganismen (DSM), on May 27th, 1993, under the number 8322, and the number 8323, respectively.

Plasmid pJK355 and pJK356 are represented by SEQ ID NO: 5 and SEQ ID NO: 6, respectively, and contain the nucleotide sequence delimited by the nucleotide located at the position 781 and the nucleotide located at the position 2423 of the sequence represented by SEQ ID NO: 1, and which is delimited by the nucleotide located at the position 1 and the nucleotide located at the position 1643 of the sequences represented by SEQ ID NO: 5 and SEQ ID NO: 6.

Vectors according to the invention are more particularly characterized in that they contain in one (or several) of their site(s) non essential for their replication one (or several) selectable food grade marker gene, and/or heterologous sequence(s) coding for a predetermined polypeptide.

Said predetermined polypeptide can be chosen among polypeptides produced by species and strains susceptible to be used as starter organisms in fermentation processes, and more particularly lactic acid bacteria, such as *Lactococcus*, *Streptococcus* and *Lactobacillus*, susceptible to produce aminopeptidase such as X-Prolyl-dipeptidyl-aminopeptidase (PepX) from *Lactobacillus delbrückii* ssp. *lactis or* polypeptides derived thereof.

The invention also relates to cellular hosts transformed by a vector according to the invention.

Cellular hosts according to the invention comprise the regulation elements enabling the expression of the nucleotide sequence coding for a protein according to the invention in these hosts.

Said cellular hosts are more particularly chosen among those generally recognized as safe for human or animal organisms, such as lactobacilli.

The invention also relates to a process for the production of a predetermined polypeptide characterized in that it comprises the following steps:
- the culture in an appropriate medium of a cellular host such as described above transformed with a vector according to the invention comprising a sequence coding for the predetermined polypeptide,
- the recovery of the predetermined polypeptide produced by the above said cellular host from the above said culture medium,
- the purification of the polypeptide thus obtained.

The invention also relates to fermentation processes comprising the culture in an appropriate medium of a cellular host, such as described above, transformed with a vector according to the invention comprising a sequence coding for a predetermined polypeptide.

The invention also relates to a process for the preparation of fermented foodstuff, and more particularly of sausage or cheese, which comprises a step of treatment of food material to be fermented, such as milk (cow, buffalo, sheep milk for example), with an appropriate amount of at least one of the cellular host according to the invention transformed with a vector comprising a sequence coding for a polypeptide susceptible to be active in fermentation processes.

This process could also comprise a step of treatment of food material to be fermented, such as milk, with other species and strains susceptible to be used as starter organisms in fermentation processes, and more particularly lactic acid bacteria, such as *Lactococcus*, *Streptococcus* and *Lactobacillus*, susceptible to produce an aminopeptidase useful in fermentation processes, such as PepX protein.

The processes above described can be used as fermentation processes for the obtention of hard cheeses, such as Emmentaler type cheese.

The invention also relates to foodstuff, and more particularly cheese, such as obtained by the methods described above.

The invention also relates to a process for preparing the proteins of 56, 221, and 146 amino acids according to the invention, comprising the following steps:
- the culture in an appropriate medium of a cellular host as described above comprising the nucleotide sequence coding for one of these proteins,
- the recovery of the polypeptide produced by the above said cellular host from the above said culture medium,
- the purification of the polypeptide produced, more particularly according to the method above described.

The polypeptides of the invention can also be prepared according to the classical techniques in the field of peptide synthesis.

The synthesis can be carried out in homogeneous solution or in solid phase.

For instance, the synthesis technique in homogeneous solution which can be used is the one described by Houbenweyl in the book entitled "Methoden der organischen Chemie" (Method of organic chemistry) edited by E. Wünsch, vol. 15-I and II, THIEME, Stuttgart 1974.

The polypeptides of the invention can also be prepared in solid phase according to the methods described by Atherton and Sheppard in their book entitled "Solid phase peptide synthesis" (IRL Press, Oxford, New York, Tokyo, 1989).

The invention also relates to a process for preparing the nucleic acids according to the invention.

A suitable method for chemically preparing the single-stranded nucleic acids (containing at most 100 nucleotides of the invention) can be carried out according to the using the automatic β-cyanoethyl phosphoramidite method of DNA synthesis described in Bioorganic Chemistry 4; 274-325 (1986).

In the case of single-stranded DNA, the material which is obtained at the end of the DNA synthesis can be used as such.

A suitable method for chemically preparing the double-stranded nucleic acids (containing at most 100 bp of the invention) comprises the following steps:
- DNA synthesis of one sense oligonucleotide using the automatic β-cyanoethyl phosphoramidite method above cited, and DNA synthesis of one antisense oligonucleotide using either the above-mentioned automatic β-cyanoethyl phosphoramidite method, or enzymatic transcription of the sense-strand using a specific primer hybridizing to the 3'-end of the sense strand,
- combining the sense and antisense oligonucleotide by hybridization in order to form a DNA duplex,
- cloning the DNA duplex obtained into a suitable plasmid vector and recovery of the DNA according to classical methods such as restriction enzyme digestion and agarose electrophoresis, or by PCR amplification according to the procedure outlined above.

A method for the chemical preparation of nucleic acids with lengths greater than 100 nucleotides - or base pairs, in the case of double-stranded nucleic acids - comprises the following steps:
- assembling the synthesized oligonucleotides, provided at their ends with different restriction sites, the sequences of which are compatible with the succession of amino acids in the natural peptide, according to the principle described by Urdea et al. in Proc. Nat. Acad. Sci. USA 80; 7461-7465 (1983),
- cloning the DNA thereby obtained into a suitable plasmid vector and recovery of the desired nucleic acid according to classical methods such as restriction enzyme digestion and agarose gel electrophoresis.

Other characteristics and advantages of the invention will appear in the following examples and the figures illustrating the invention.

### I) Figure legends

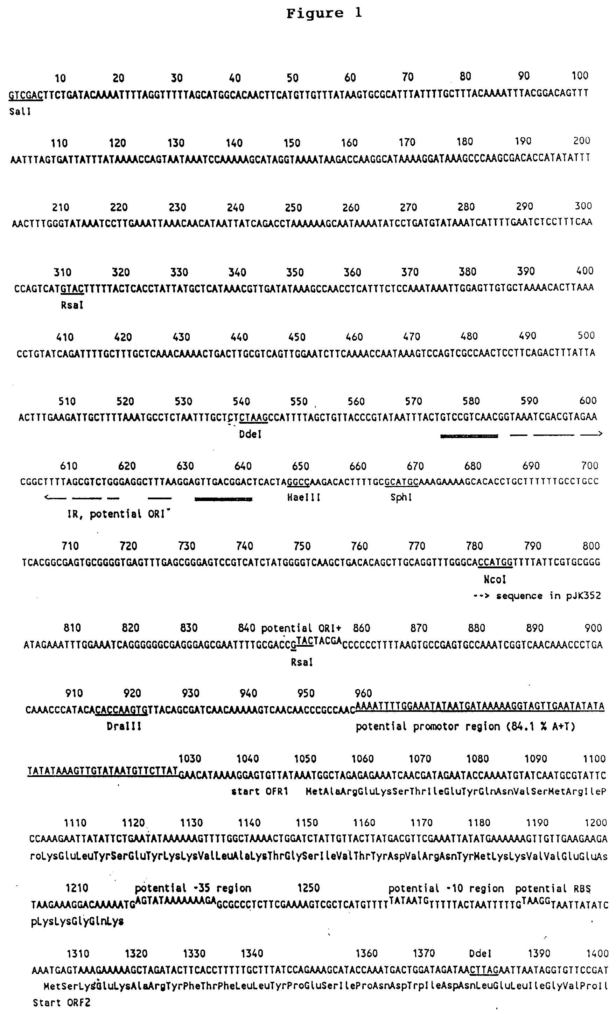

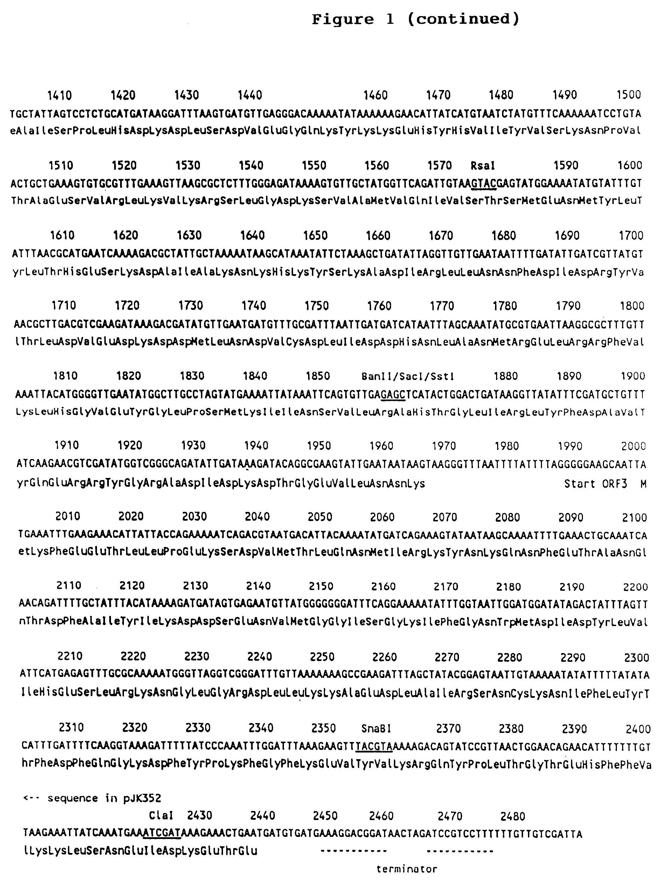

Figure 1. Nucleotide sequence of plasmid pLC2. The deduced amino acid sequences for the three open reading frames are given below the non coding strand. Numbering starts with +1 at the first nucleotide of the *Sal*I site, used for cloning of the complete plasmid in vector pJK80. The nucleotides subcloned for construction of the vectors pJK352, pJK353, pJK354, pJK355 and pJK356 are marked. A possible plus *ori* is indicated. The inverted repeat (IR) between bps 574 and 640, proposed as minus *ori*, is underlined, with its highly conserved stem structure double underlined. The potential promoter sites upstream ORF1 and ORF2 as well as the positions of some pertinent restriction sites are shown. A stem-loop with a free energy value dG of -15.2 kcal/mol, followed by a stretch of T residues, which could be located downstream of ORF3 (bps 2448-2475) might function as a translational terminator, as predicted by the computer algorithm of Brendel and Trifonov (1984).
   The sequences have been deposited in the EMBL, GenBank and DDBJ databases under accession number Z14234.

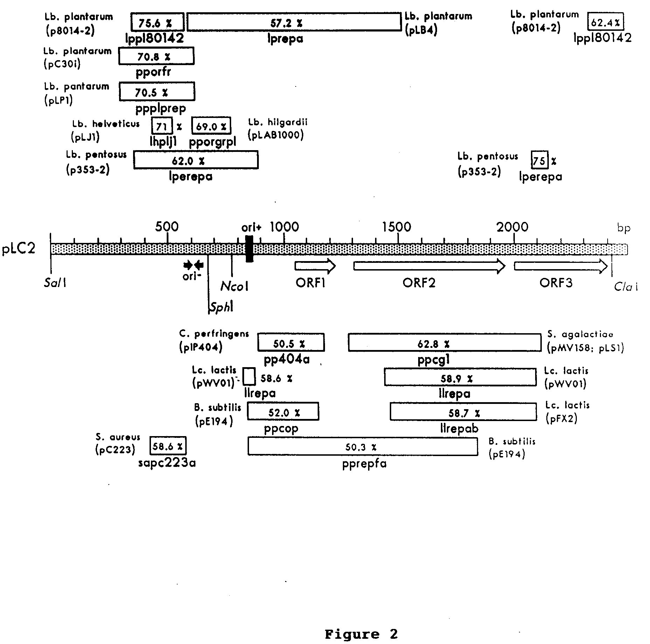

Figure 2: Schematic alignment of pLC2 nucleotide sequence with homologous regions of plasmids from Gram bacteria. Sequence homology resulted from searching pLC2 against the complete EMBL database with the programs FASTA (Pearson and Lipman, 1988) and BLASTN (Altschul et al., 1990). The stretches of homology, drawn to scale, are given as boxes with the EMBL entry name below. The bacterial host names of the different plasmids (names in brackets) are placed beside, and the degree of sequence identity resulting from FASTA analysis, inside the boxes. The complete plasmid pLC2 was searched against the database, thus resulting in maximum stretches of high homology. If the searched sequence is limited (e.g. isolated ORFs) different values are obtained. pLC2 with some pertinent features is given in the center of the drawing.

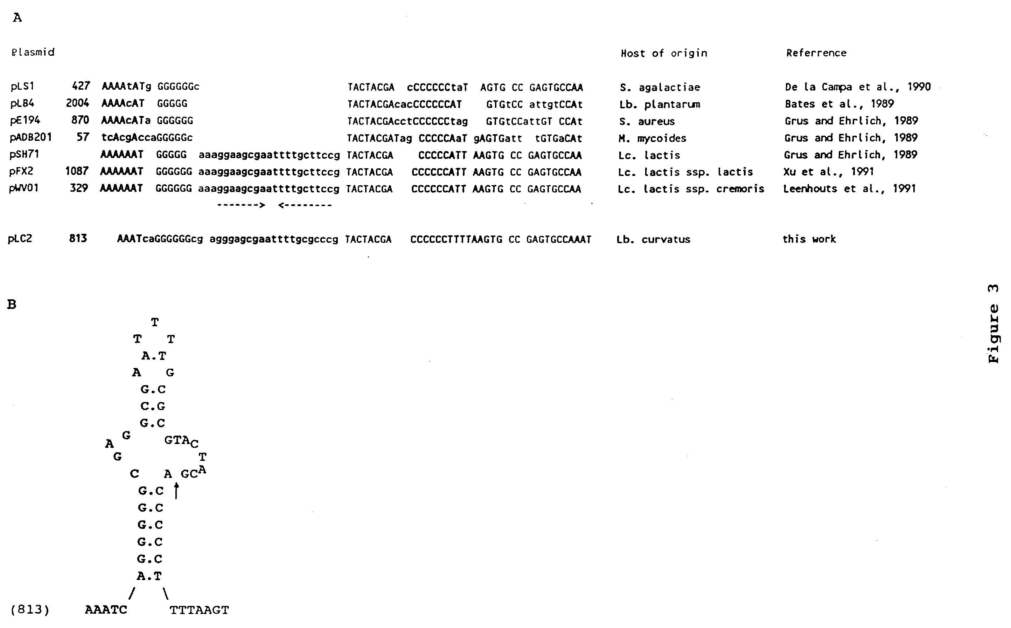

Figure 3: Comparison of plasmid plus *ori s*ites.
   A) A conserved inverted sequence of 23 bp in pFX2 and pSH71 is marked by facing arrows (Xu et al., 1991). A similar sequence but having less potential to form a stem-loop structure, can be located in pLC2 at the same position.
   B) A possible loop structure in the putative plus *ori* locus of pLC2 with a free energy of - 20.8 kcal/mol. The putative nicking site, as found in pLS1 is indicated by an arrow.

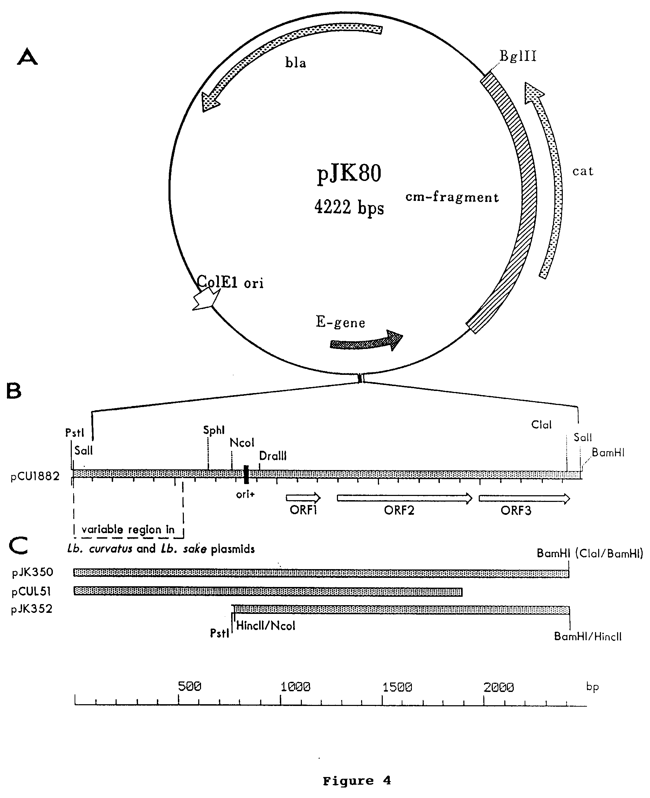

Figure 4: Determination of the minimal replicon of pLC2.
   A) Cloning of pLC2 as *Sal*I fragment in pJK80 (for details see bacterial strains and plasmids) resulted in pCU1882.
   B) the variable region, described by Vogel et al. (1991) in the noncoding part of the plasmid is indicated.
   C) All the plasmids presented replicate in lactobacilli. pJK350 arose from pCU1882 by deletion of a 75 bp *Cla*I/*Bam*HI fragment. Exonuclease III treatment of pCU1882, linearized with *Cla*I, resulted in pCUL51, with a deletion reaching into ORF2 (position 1909) as examined by sequence analysis. Plasmid pJK352 was obtained after subcloning the *Nco*I/*Bam*HI fragment from pJK350, treated with Klenow enzyme, into the *Hinc*II site of pJK80.

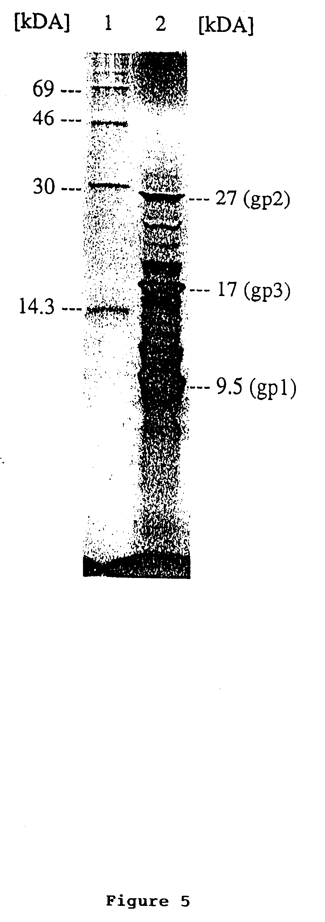

Figure 5: Autoradiogramm of the [³⁵S] methionine labelled proteins produced by *in vitro* transcription/translation and separation by SDS-PAGE (15 % acrylamide). The gene products (gp) proposed to be encoded by the three open reading frames are labelled gp1, gp2, and gp3 respectively. Molecular masses of [¹⁴C]-methylated protein markers are indicated.

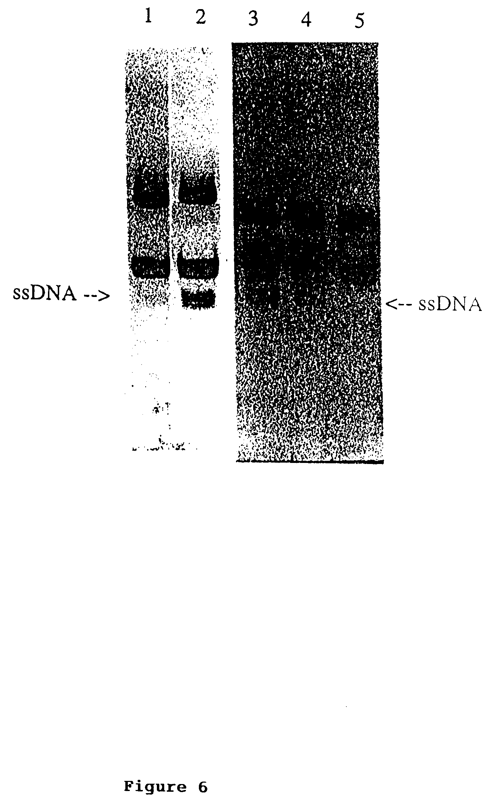

Figure 6: Whole cell lysates of *Lb. curvatus* LTH683 hybridized with digoxigenin labelled pLC2 probe. A faint ssDNA band detectable in untreated cells (lane1) accumulated after inhibitin with rifampicin (lanes 2). Prior treatment with S1 nuclease, DNA was precipitated with ethanol, washed and resuspended in the original volume. 0 units S1 (lane 3), 0,4 units (lane 4), 4 units (lane 5).
Figure 7: (A) Construction of cloning vectors pJK355 and pJK356.
   pCU1882 contains the complete plasmid pLC2 (shaded box) in pJK80 (open box). The antibiotic resistance genes (cat: chloramphenicol transcetylase, and bla: beta lactamase) are indicated by dotted arrows.
   Plasmid pJK352 deleted for the "variable DNA region" (Fig. 4) was commencing product for vector construction.
   pJK352d served as a control, confirming that deletion of ColE1 had no effect on replicational properties.
   The synthesized polylinker fragment was integrated into the *Pst*I site of pJK352 and the two possible orientations resulted in pJK353 and pJK354.
   For the deletion of the ColE1 origins pJK353 was restricted with *Bgl*II and *Ava*I, ends were filled in with Klenow enzyme and religated. The resulting plasmid pJK355 retained the *Bgl*II and *Ava*I sites.
   pJK354 was cut with *Bgl*II and *Xba*I, blunt ends created with Klenow enzyme, and religated. The construct pJK356 had lost the restriction sites for *Bgl*II and *Xba*I.
   (B) Nucleotide sequence of the multiple cloning sites in plasmids pJK353, pJK354, pJK355, and pJK356, with orientations corresponding to Fig. 5 A.

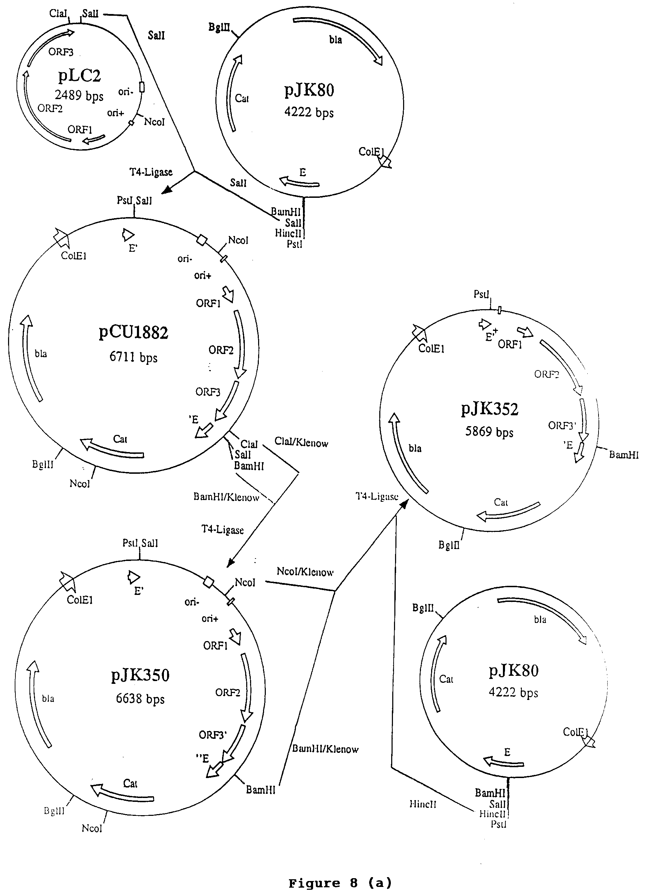

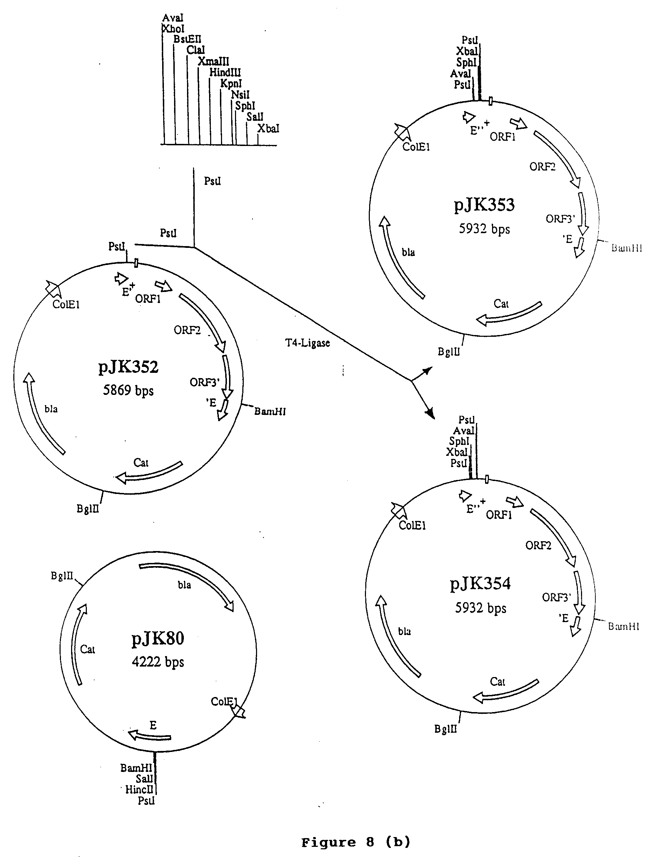

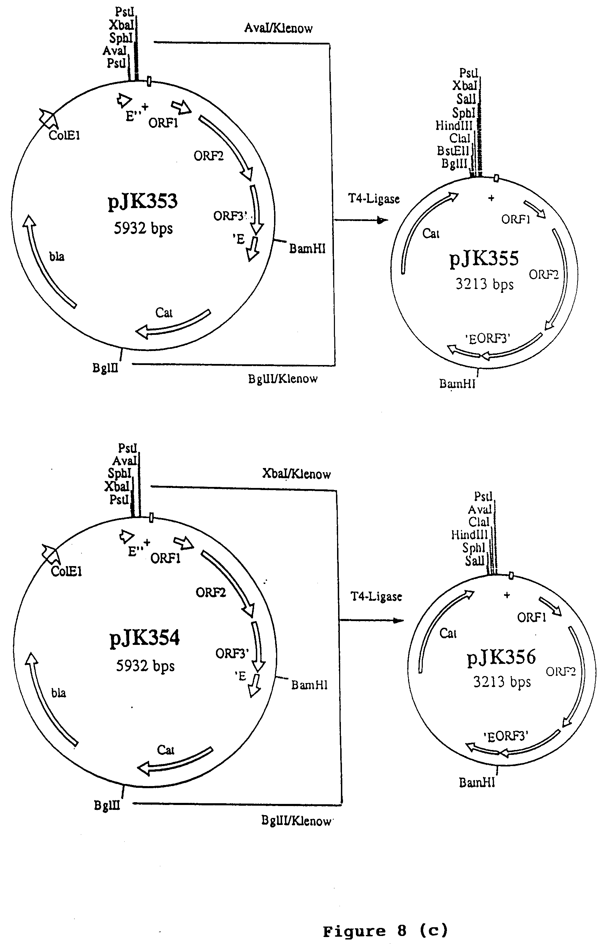

Figure 8 (a, b, and c) construction of cloning vectors pJK355 and pJK356:
   The cryptic plasmid pLC2 was linearized with *Sal*I and ligated into vector pJK80 cut with *Sal*I. Ligation in plasmid pCU1882 which contains the complete cryptic pLC2. The antibiotic resistance genes of the vector (cat: chloramphenicol transacetylase, and bla: beta lactamase), the ColE1 origin of replication, the lysis gene E of bacteriophage φX174, as well as the three open reading frames (ORF) of pLC2 and the minus and plus origins are indicated.
   pCU1882 WAS restricted with *Cla*I and *Bam*HI, then treated with Klenow enzyme to produce blunt ends, and ligated. The resulting plasmid PJK350 was deleted for the 75 bps *Bam*HI/*Cla*I fragment.
   pJK350 was cut with *Nco*I and *Bam*HI. The 1643 bps fragment was isolated, treated with Klenow enzyme and ligated into the vector pJK80 cut with *Hinc*II. The resulting plasmid pJK352 deleted for the "variable DNA region" (Fig.4) was commencing product for vector construction.
   The synthesized polylinker fragment with flanking *Pst*I sites was integrated into the PstI site of pJK352 and the two possible orientations resulted in pJK353 and pJK354 respectively.
   For the deletion of the ColE1 origins pJK353 was restricted by *Bgl*II and *Ava*I, ends were filled in with Klenow enzyme and religated. The resulting plasmid pJK355 retained the *Bgl*II and *Ava*I sites.
   pJK354 was cut with *Bgl*II and *Xba*I, blunt ends created with Klenow enzyme, and religated. The construct pJK356 had lost the restriction sites for *Bgl*II and *Xba*I.

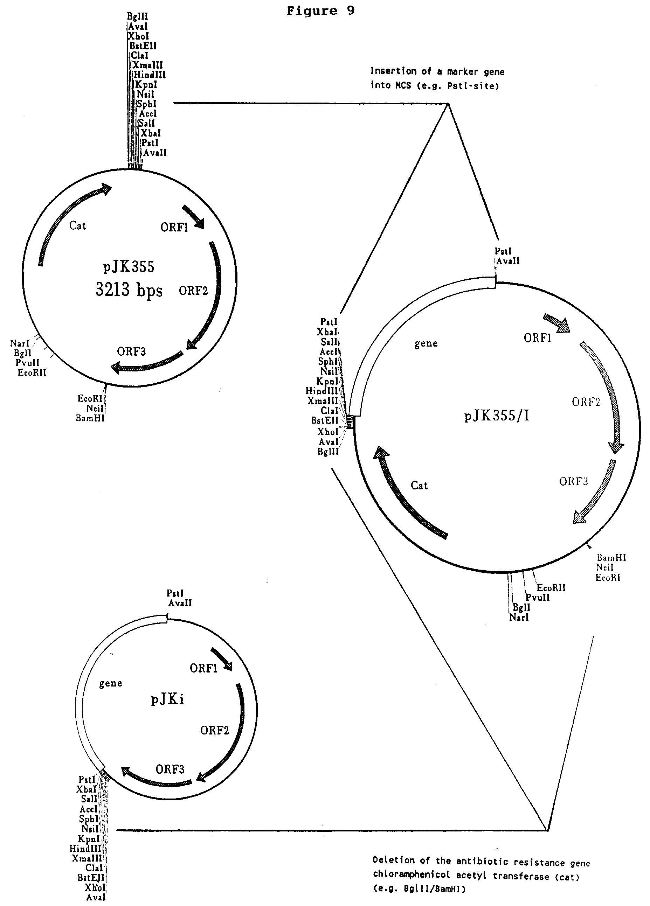

Figure 9: Replacement of the chloramphenicol resistance gene in pJK355 with a selectable food grade marker gene.

All restriction sites listed are unique. The MCS of either pJK355 or pJK356 can be used to insert a gene in that way that the *cat* gene can be eliminated by restrincting the plasmid with a pair of single cutting enzymes followed by recircularization.

### II) Materials and methods

### 1) Bacterial Strains, Plasmids, and Growth Conditions

The bacterial strains used are summarized in Table 1. *Escherichia coli* and *Bacillus subtilis* were grown at 37°C in Luria broth (Miller, 1972), *Lactococcus lactis* was propagated in M17 supplemented with 0.5 % glucose (GM17) (Terzaghi and Sandine, 1975), and lactobacilli in MRS (De Man et al., 1960) at 30°C. Ampicillin and chloramphenicol were added to concentrations of 100 and 10 µg/ml, respectively. Plasmid pJK80 is a positive selection vector for insert DNA based on pUH84 (Henrich and Plapp, 1986), with a chloramphenicol resistance (Cm^{R} ) gene as marker in Gram positive bacteria, but which is not able to replicate in Gram positives, unless a functional replicon is inserted. The Cm^{R} gene was isolated as a 1035 bp *Hpa*II/*Sau*3A fragment from pGK12 (Kok et al., 1984), treated with Klenow enzyme and ligated into the filled-in *Nde*I site of pUH84.

After integration of the functional replicational region of pLC2, the hybrid plasmid, could be selected in lactobacilli with chloramphenicol.

The cryptic plasmid pLC2 was isolated from *Lactobacillus curvatus* LC2 LTH683 deposited at the DSM under the number 8321 on May 27th, 1993.

### 2) Recombinant DNA Techniques.

Restriction enzymes and other nucleic acid-modifying enzymes were used as recommended by the manufacturers. Isolation of plasmid DNA from *E. coli* was performed as described by Sambrook et al (Sambrook et al., 1989). Plasmid DNA isolation from *Lb. casei* or *Lb curvatus* was performed as described elsewhere (Zink et al., 1991).

### 3) Inhibition of RNA polymerase by rifampicin and detection of single-stranded DNA in cell lysates of Lb. curvatus LTH683.

For accumulation of ssDNA, cultures of *Lb. curvatus* were incubated with Rifampicin as described previously (Leenhouts et al., 1991) and whole-cell lysates were prepared with slight modifications. The lysis buffer of 10 mM Tris-HCl, 1 mM EDTA, pH 8.0 was supplemented with lysozyme (10 mg/ml) and mutanolysin (500 U/ml) and cells were incubated at 37°C for 15 min. Lysates were incubated with Sarkosyl for 15 min at 60°C. After phenol extractions crude lysates were suspected to agarose gel electrophoresis for detection of ssDNA accumulation. Prior nuclease S1 treatment the DNA was precipitated and washed with 80 % ethanol. After electrophoresis in a agarose slab, the DNA was transferred to a nylon membrane (Hybond, Amersham). The presence of ssDNA was determined by Southern hybridization with digoxigenin-dUTP labelled pLC2 as probe. Labelling and detection of the DNA was carried out using the nonradioactive digoxigenin kit (Boehringer, Mannheim, FRG).

### 4) Cloning of the pLC2 origin in pJK80

The unique *Sal*I site of the cryptic plasmid pLC2 was used to produce a linear molecule of 2.5 kb in size. Following the strategy described earlier (Zink et al., 1991), we were able to clone this fragment with the functional origin into the *Sal*I site of pJK80, thus inactivating the lysis gene E. Hybrid plasmids were isolated from *E. coli* and introduced into *Lb. casei* LK1 by electroporation. The chloramphenicol resistance marker of plasmid pJK80, originating from pC194 (Horinouchi and Weissblum, 1982) served as a selectable marker in lactobacilli. Plasmid pCU1882 could then be isolated from chloramphenicol resistant transformants.

### 5) DNA sequence analysis

For nucleotide sequencing of inserts in pJK80, a pair of universal sequencing primers, adjacent to the multiple cloning site, was synthesized. Synthetic oligonucleotide primers deduced from the investigated sequence, were synthesized and allowed direct sequencing of double stranded pLC2 plasmid DNA. This proofed that the whole plasmid pLC2 is represented by the *Sal*I restriction fragment cloned in vector pJK80.

The DNA sequence of each strand was determined using the T7 DNA polymerase sequencing kit (Pharmacia, Uppsala, Sweden), which is based on the dideoxynucleotide chain termination method (Sanger et al., 1977) in the presence of [α- ³⁵S] dATP (Amersham). For computer-assisted sequence analysis the Microgenie (Beckman, Palo Alto, CA), PC-Gene (IntelliGenetics, East El Camino Real, CA), and HUSAR (GENIUSnet, Heidelberg) software were used. Sequencing primers were synthesized using Applied Biosystems Model 392 DNA synthesizer and reagents.

### 6) Transformations

*Lb. casei, Lb. curvatus, Lactococcus lactis* and *E. coli* cells were transformed by electroporation using a Bio-Rad Gene Pulser (Bio-Rad Laboratories, Richmond, CA) as described before (Zink et al., 1991; Vos et al 1989; Dower et al., 1988). Preparation of competent *B. subtilis* cells and transformation with plasmid DNA was performed according to the 'Groningen Method' (Bron, 1990).

### 7) DNA-directed in vitro transcription:translation and Sodium dodecyl sulfate polyacrylamide gel electrophoresis (SDS-PAGE) electrophoresis.

*In vitro* transcription and translation was performed with plasmid DNA purified by CsCl density gradient centrifugation, using a cell-free coupled transcription-translation system derived from *E. coli* (Amersham). Synthesized proteins labelled with L-[³⁵S] methionine were separated by SDS-PAGE on vertical slab gels using the Laemmli (1970) buffer system and detected by autoradiography.

### 8) Determination of plasmid stability

Strains LK1 and LTH1432 transformed with plasmids pCU1882, pJK355 and pJK356 were incubated without selective pressure. After reaching the stationary phase the culture was diluted by 1:16384 for a further cycle, thus reaching approx. 14 generations per cycle. Loss of plasmid was measured by counting the survivals plated on agar plates with and without the selective antibiotic chloramphenicol. For determination of plasmid content and plasmis integrity, DNA preparations of chloramphenicol resistant colonies were examined by electrophoresis.

### 9) Host Range of pLC2 replicon

For determination of hostrange, the strains listed in Table 1. were transformed with plasmids pJK351, pJK355 and pJK356, having only the unique origin of pLC2, and selected for resistance against chloramphenicol (10µg/ml; *Lc. lactic* MG1363 was selected with 20 µg/ml). In *E. coli* resistance had to be induced in subinhibitory levels (500 ng/ml) (Horinouchi and Weisblum, 1982). Transformed colonies were grown to stationary phase in liquid culture and subjected to plasmid isolation.

### II RESULTS

### A) CHARACTERIZATION OF THE CRYPTIC PLASMID pLC2

### 1) General features of the nucleotide sequence.

The G+C content of the cryptic *Lactobacillus* plasmid pLC2 was 33.6 %, which is low in respect to the 43 % reported for the chromosome (Dellaglio et al., 1973). Computer assisted analysis revealed the presence of three open reading frames, potentially capable of encoding polypeptides with molecular sizes of 6633, 25688 and 17210 D (Fig. 1).

The establishment of the pLC2 sequence and the determination of the essential replicational region enabled the construction of pJK355 and pJK356 to be used as small cloning vectors with multiple cloning sites.

### 2) Nucleotide sequence homologies

The complete nucleotide sequence of pLC2 was searched against the EMBL database, with the result that the major part of the plasmid has a significant degree of homology in numerous plasmids from Gram positive bacteria which are all replicating by a RCR mechanism. The degree of similarity on the nucleotide level is summarized in figure 2, which demonstrates that almost the entire pLC2 plasmid has homology to plasmids which have themselves origins of distinct groups. Long stretches of homology, discussed below in more detail, were found for the regions containing the proposed plus and minus origins and for the ORF1 and ORF2 stretches. Only few homologous sequences were found for the nonessential and delectable regions of pLC2.

### 3) The pLC2 plus ori+ site

By comparing the sequence with three types of plus origin sequences pT181, pC194 or pE194 (Gruss and Ehrlich, 1989), a region homologous to the plus ori sites of the pE194 group plasmids was identified (Fig. 3 A). The location of the ori site upstream and not within the proposed protein coding regions in pCU1882 (Fig. 4) was confirmed by deleting the 924 bp *Pst*I-*Dra*III fragment, thus making the plasmid nonfunctional in *Lactobacillus casei* LK1. The proposed ori+ (position 813-869), which is the palindromic sequence with the highest free energy in pLC2, possesses an internal stem-loop structure (Fig. 3 B), which could be the signal for plasmid replication as it has been shown for plasmid pLS1 (de la Campa et al., 1990).

### 4) The pLC2 minus origin

Other secondary replication functions in ssDNA plasmids are the minus origins (MO) of the replication, palindromic sequences functioning as initiation sites for lagging strand synthesis. They are known to be located within regions of dyed symmetry (Bron et al., 1988; Novick, 1989). This site is normally located outside the minimal replication region, and its deletion may influence stability and copy number (Gruss and Ehrlich, 1989). Different sequence representing the lagging-strand initiation site have already been characterized: pa1A (Gruss et al., 1987), BA3 (Bron et al., 1988), BAA1 (Devine et al., 1989) and Leer et al., recently described a new type not belonging to these groups, which is highly conserved in plasmids like p8014-2, pC30il, pLB4 and pLJ1 (Leer et al., 1992).

Since typical minus origins as palindromic sequences of up to 200-300 base pairs in length have the potential to form secondary hairpin structures, the plC2 sequence was searched for such regions. The proposed ori plus site with a delta G of -20.8 kcal/mol was found, but using the available computer software, only weak (< -15 kcal /mol), short (approx 10 bp), and imperfect structures could be additionally localized, which did not resemble a typical minus origin. But if the results from homology comparison with other plasmids were examined carefully, a region upstream the pLC2 ori+ site, which is not coding for proteins shows strong homology to *Lb. plantarum and Lb. pentosus* and *Lb. helveticus* plasmids (Fig. 2). Between bps 305 and 640, pLC2 has more than 70 % identity to the *Lb. plantarum* plasmids pLP1 (Bouia, et al., 1989), pC30il (Skaugen, 1989), p8014-2 (Leer et al. 1992), pLB4 (Bates and Gilbert, 1989), to the *Lb. pentosus* plasmid p353-2 (Leer et al. 1992) and to the plasmid pLJ1 from *Lb. helveticus*. As reported by Leer et al. (1992) these plasmids contain a new type of minus ori, which is just in the region showing homology to pLC2. Based on these data an imperfect stem-loop structure with a free energy of -20 kcal/mol could be calculated. This free energy and the size of the structure is in the range of the values of the inverted repeats (IR) which were found for plasmids having the new type of minus origin. The stem of the IR is almost perfectly conserved (double underlined in Fig. 1) whereas the loop with weaker base pairing is not. Upstream of this IR there is a longer stretch of homology (position 305-640 of the pLC2) to other plasmids. Leer et al (1992) have found that the difference in size between the closely related *Lb. plantarum* plasmid (p8014-2) and the *Lb. pentosus* plasmid p353-2 is an effect of variability in this region upstream of ori+. This notice agrees well with the observation made by Vogel et al. (1992) after comparative restriction analysis of different *Lb. sake* and *Lb. curvatus* plasmids. They noticed a highly conserved and a variable region for all plasmids (including pLC2) observed and the sequencing data obtained from pLC2 now allow to appoint this region (Fig. 4). It is identical to the DNA stretch with the IR capable to be the MO and the region upstream which might have regulatory functions or incompatibility properties but which is not coding for proteins.

### 5) Protein coding regions

Downstream of the ori site, three open reading frames ORF1 (position 1049-1216), ORF2 (position 1303-1965) and ORF3 (position 2000-2437) were located, with 56, 221, and 146 codons respectively (Fig. 1). The method developed by Kolaskar and Reddy (1985) allowed the prediction for all three reading frames to have high coding probabilities. Possible coding regions could not be located on the lower strand of the plasmid.

ORF1 is proceeded upstream by a 69 bp sequence of 84.1 % A+T (958-1026), which could serve as -35 and -10 sequence of a promoter, followed by a potential ribosome-binding site (RBS) aAGGGA at position 1036. ORF1 shows homology to the cop protein of the *S. aureus* plasmid. pE194 (Byeon and Weisblum, 1990). Twelve bps upstream the ATG site of ORF2, a potential RBS (TAAGG), proceeded by a promoter -10 sequence TATAAT can be localized. We have still to await if expression signals of lactobacilli have the same features conserved as other Gram positive bacteria and further investigations have to be made to determine what kind of changes in primary sequences effect the efficiency of gene expression.

Searching the nucleotide or deduced amino acid sequence of ORF2 against a data base, resulted in homologies to the replication proteins of the pE194 group plasmids, which agrees to the classification of the pLC2 ori + sequence being the corresponding target site of the rep protein. The complete gene product of ORF2 has 59.9 % homology to the replication protein of pMV158 or pLS1 and 48.1 % identity to repA of plasmid pWVO1.

Interestingly the plasmids which have a similar MO as pLC2 do not belong to the pE194 class but have ori+ and corresponding *rep* genes as the pC194 type RCR plasmids. This favours the theory of Josson et al. (1990) that plasmids might consist of functional cassettes, which can be exchanged by horizontal transfer through the different genera.

### 6) In vitro transcription/translation assay

Evidence that the predicted protein coding regions ORF1, ORF2, and ORF3 are encoding, was obtained by coupled *in vitro* transcription and translation of plasmid pLC2. The plasmid was shown to direct the synthesis of proteins with molecular weights of approximately 27, 17, and 9.5 kDa (Fig. 5). These products agree quite well with the predicted sizes of 25.688 (ORF2), 17.210 (ORF3), and 6.633 (ORF1). Nevertheless it should be kept in mind that genes of *Lb. curvatus* are transcribed and translated by an *E. coli* derived system and heterologous genes are not expected to be always well expressed.

### 7) Replication of pLC2 via a RCR mechanism

The sequence homology between the pLC2 plus origin to the pE194-type plasmids, as well as the imperfect repeat (bps 594-640) to a minus origin and the similarity of the protein coding regions to other Gram positive plasmids, suggested that pLC2 is like all these other related plasmids replicating by a RCR mechanism. To proof the evidence, whole cell lysates of LTH683 were electrophoretically separated and intermediate DNA was detected after hybridization with a digoxgenin labelled pLC2 probe. As demonstrated in Fig. 6 only a faint ssDNA brand could be detected. The proof that the fast running DNA was single stranded could be performed by digestion with nuclease S1. It is generally believed that conversion to dsDNA is initiated by host RNA polymerase (Gruss and Ehrlich, 1989) and that the leading strand is primed by antisense RNA. Therefore growing cells were inhibited with rifampicin, an inhibitor of RNA polymerase and after incubation with rifampicin, ssDNA significantly accumulated. The very low amount of ssDNA in untreated cells suggested that ssDNA is very efficiently converted into dsDNA. The occurence of a ssDNA intermediate reinforces the opinion that pLC2 replicates via a RCR mechanism.

### 8) The essential region of pLC2 necessary for replication

Deletion derivatives of pCU1882 were constructed and tested for autonomous replication in lactobacilli. The essential region for plasmid pLC2 (as summarized in Fig. 4) could be located within basepairs 780 (*Nco*I site in pJK352) and 1909, being the termination site of exonuclease III deletion in pCUL51. Therefore the genes coded by ORF1 and ORF2 were sufficient for autonomous replication. Additionally the IR (bps 574-640 in pLC2) proposed for being the minus origin is non essential for replication. The comparative studies on small cryptic plasmids from *Lb. curvatus* and *Lb. sake*, by Vogel et al (1991) revealed a variable region among the different plasmids and this variable stretch in pLC2 corresponds to the deletable *Sal*I/*Sph*I fragment (bps 1-664) removed in pJK352 and all its derivatives (Fig. 4). This may suggest an increased instability accompanied with accumulation of ssDNA but this has to be further investigated in detail.

### B) CONSTRUCTION OF LACTOBACILLUS CLONING VECTORS

The sequence of plasmid pCU1882, containing the whole cryptic plasmid pLC2 as *Sal*I fragment in the vector pJK80, could be deduced from the established pLC2 nucleotide sequence and the vector sequence. The deletion derivative pJK352 was chosen for the construction of vectors with multiple cloning sites. Since pJK352 contains two replicons, the pLC2 origin and ColE1, the latter should be deleted after insertion of a polylinker fragment. In advance it has been shown, that the deletion of the ColE1 replicon in pJK352d (Fig. 7A) had no effect on the ability of replication in lactobacilli.

A multiple cloning site was designed for pJK352 by choosing rare cutting restriction enzymes sites not present in the plasmid. Two complementary single stranded 73 mers were synthesized (Fig. 7B) and annealed to double stranded DNA. *Pst*I ends were generated with the appropriate restriction enzyme and ligated into the phosphatase treated *Pst*I site of pJK352. Transformation of the ligase mix and isolation of plasmids was carried out in LK1. The two possible orientations of the polylinker fragment could be determined by examining the DNA fragments produced by restriction with *Ava*I/*Bst*XI, and resulted in plasmids pJK353 and pJK354. The ColE1 origins of these plasmids were removed (Fig. 7A), resulting in the vectors pJK355 and pJK356.

### 1) Plasmid stability

For application in industrial fermentation processes, it is in many cases desirable that the vector is stably maintained in the host cell, even in non selective medium. Transformers of LK1 with plasmids pCU1882, pJK355 and pJK356 were incubated without selective pressure. No significant loss of plasmid could be detected after incubationfor 56 generations (>90 % cells contained plasmid), but after an additional transfer (equivalent to 70 generations) only 75 %, and after another transfer (84 generations), the plasmids pJK355 and pJK356 were lost to 40 %. The content of pCU1882 remained unchanged. This segregational instability, probably a result from deleting the ori-, was not accompanied by structural instability. The minimisation of the replicon lead to a slight loss of stability but because in many food fermentation processes the inoculum with starter cultures is very high and bacteria undergo only few generations (Vogel 92 et al., 1992), the cloning vectors constructed may be useful in lactobacilli.

### 2) Host range

After transformation of plasmids pJK352d and pJK355 or pJK356 into the heterologous Gram strains *Bacillus subtilis* 6GM and *Lactococcus lactis* ssp. *lactis* MG1363, chloramphenicol resistant colonies could be propagated in liquid culture and plasmid DNA isolated was unchanged in its electrophoretic pattern. Transformation of *E. coli* cells resulted in small chloramphenicol resistant colonies, but no growth was obtained in liquid medium. This indicated for non successful replication of the lactobacillal replicon in *E. coli.* Selection with chloramphenicol was possible as demonstrated with pCU1882 and other derivatives having both origins pLC2 and ColE1. But all these plasmids had a tendency to delete DNA fragments after several passages in *E. coli,* whereas in *Lactobacillus* this phenomena was not observed. This observation suggested that ColE1 sequences are not expressed in lactobacilli and do not impair the segregational stability, whereas in *E. coli* the lactobacillal genes or structures are interfering with the replication mechanism of the *E. coli* vector

**Table 1**

| Bacterial strains | | |
|---|---|---|
| Strain | (formely referred to as) | Reference |
| *Lactobacillus casei* LK1 | (WS97) | Zink et al., 1991 |
| *Lactobacillus curvatus* LTH683 | (Lc2) | Vogel et al., 1992 |
| *Lactobacillus curvatus* LTH1432 | (Lc2-c) | Vogel et al., 1992 |
| *Lactococcus lactis ssp. lactis* MG1363 | | Gasson, 1983 |
| *Bacillus subtilis* 6GM | | Haima et al., 1990 |
| *Escherichia coli* K-12 MM294 | | Hanahan, 1983 |

### REFERENCES

Altschul et al., (1990), J. Mol. Biol. **215**, 403-410.
Bates E.E.M. and Gilbert, H.J. (1989), Gene **85**, 253-258.
Bouia et al., (1989), Plasmid **22**, 185-192.
Brendel V. and Trifonov E.N. (1984), Nucleic Acids Res. **12**, 4411-4427.
Bron S. (1990), Plasmids. In: Harwood C.R. and S.M. Cutting (ed) Molecular Biological Methods for Bacillus. 'A Wiley-Interscience publication', Chichester, West Sussex, England, pp 75-174.
Bron et al., (1988), Plasmid **19**, 231-241.
Byeon W-H. and Weisblum B., (1990), J. Bacteriol. **172**, 5892-5900.
Cocconcelli et al., (1991), Research in Microbiology **142**, 643-652.
De la Campa et al., (1990), J. Mol. Biol. **213**, 247-262.
Dellaglio et al., (1973), J. Gen. Microbiol. **74**, 289-297.
De Man et al., (1960), J. Appl. Bacteriol. **23**, 130-135.
Devine et al., (1989), J. Bacteriol. **171**, 1166-1172.
Dower et al., (1988), Nucleic Acids Res. **16**, 2127-2145.
Gaier et al., (1990), Lett. Appl. Microbiol. **11**, 81-83.
Gasson M.J., (1983), J. Bacteriol. **154,** 1-9.
Gruss et al., (1987), Proc. Natl. Acad. Sci. USA **84**, 2165-2169.
Gruss A. and Ehrlich S.D., (1988), J. Bacteriol. **170**, 1183.
Gruss A. and Ehrlich S.D., (1989), Microbiol. Rev. **53**, 231-241.
Haima et al., (1990), Gene **86**, 63-69.
Hanahan D., (1983), J. Mol. Biol. **166**: 557-580.
Henrich B. and Plapp R., (1986), Gene **42**, 345-349.
Horinouchi S. and Weisblum B. (1982), J. Bacteriol. **150**, 815-825.
Jannière et al., (1990), Gene **87**, 53-61.
Josson et al., (1989), Plasmid **21**, 9-20.
Josson et al., (1990), J. Bacteriol. **172**, 3089-3099.
Kolasar A.S. and Reddy B.V.S., (1985), Nucleic Acids. Res. **13**, 185-194.
Kok et al., (1984), Appl. Environ. Microbiol. **48,** 726-731.
Knauf et al., (1992), Appl. Environ. Microbiol. **58**, 832-839.
Laemli U.K., (1970), Nature, **227**: 680-685.
Leblanc D.J. and Lee L.N., (1984), J. Bacteriol. **157**, 445-453.
Leenhouts et al., (1991), Plasmid **26**, 55-66.
McKay L.L. and Baldwin K.A., (1990), FEMS Microbiol. Rev. **87**, 3-14.
Leer et al., (1992), Mol. Gen. Genet. **234**, 265-274.
Miller J.H., (1972), "Experiments in molecular genetics". Cold Spring Harbor Laboratory, Cold Spring Harbor, N.Y.
Novick R.P., (1989), Ann. Rev. Microbiol. **43**, 537-565.
Sambrook et al., (1989), Molecular cloning: "a laboratory manual", 2nd ed. Cold Spring Harbor Laboratory, Cold Spring Harbor, N.Y.
Sanger et al., (1977), Proc. Natl. Acad. Sci. USA **74**, 5463-5467.
Skaugen M. , (1989), Plasmid **22,** 175-179.
Pearson W.R. and Lipman D.J., (1988), Proc. Natl. Acad. Sci. USA **85**, 2444-2448.
Te Riele et al., (1986), Proc. Natl. Acad. Sci. USA **83**, 2541-2545.
Terzaghi B.E. and Sandine W.E., (1975), Appl. Microbiol. **29**, 807-813.
Vogel et al., (1991), FEMS Microbiol. Letters **84,** 183-190.
Vogel et al., (1992), System. Appl. Microbiol. **15,** 129-136.
Vos et al., (1989), J. Bacteriol. **171**, 2795-2802.
Xu et al., (1991), Mol. Gen. Genet. **227**, 33-39.
Zink et al., (1991), FEMS Microbiol. Letters **78**, 207-212.

## Claims

1. Nucleic acid characterized in that it comprises:
- all or part of the nucleotide sequence represented by SEQ ID NO: 1, or its complementary sequence,
- or a nucleotide sequence susceptible to hybridize with all or part of the preceeding nucleotide sequences, more particularly a nucleotide sequence which differs from the preceeding ones by addition and/or suppression and/or substitution of one or several nucleotides,
said nucleic acid, when constituting the genome of a vector, allows this latter to replicate in a cellular host.

2. Nucleic acid according to claim 1, characterized in that it comprises the nucleotide sequence represented by SEQ ID NO: 1, or fragments of said nucleotide sequence these fragments, alone or in combination with one or several other fragments of said nucleotide sequence, being still able to allow the replication of said vector.

3. Nucleic acid according to claim 1 or claim 2, characterized in that it comprises all or part of the nucleotide sequence delimited by the nucleotide located at the position 780 and the nucleotide located at the position 1909 of the nucleotide sequence represented by SEQ ID NO: 1.

4. Nucleic acid according to any one of claims 1 to 3, characterized in that it comprises all or part of the nucleotide sequence delimited by the nucleotide located at the position 813 and the nucleotide located at the position 869 of the nucleotide sequence represented by SEQ ID NO: 1, and more particularly the TACTACGA sequence located at positions 848-855, corresponding to a potential plus origin of replication.

5. Nucleic acid according to any one of claims 1 to 4, characterized in that it comprises all or part of the nucleotide sequence delimited by the nucleotide located at the position 305 and the nucleotide located at the position 640 of the nucleotide sequence represented by SEQ ID NO: 1, and more particularly the sequence located at positions 574-640, corresponding to a potential minus origin of replication.

6. Nucleic acid according to any one of claims 1 to 5, characterized in that it comprises all or part of the nucleotide sequence delimited by the nucleotide located at the position 1049 and the nucleotide located at the position 1216 of the nucleotide sequence represented by SEQ ID NO: 1, and coding for a protein of 56 amino acids represented by SEQ ID NO: 2.

7. Nucleic acid according to claim 6, characterized in that it comprises also all or part of the nucleotide sequence delimited by the nucleotide located at the position 1027 and the nucleotide located at the position 1048 of the nucleotide sequence represented by SEQ ID NO: 1, and more particularly the sequence AGGGA at positions 1036-1040, corresponding to a potential ribosome binding site.

8. Nucleic acid according to claim 6 or claim 7, characterized in that it comprises also all or part of the nucleotide sequence delimited by the nucleotide located at the position 958 and the nucleotide located at the position 1026 of the nucleotide sequence represented by SEQ ID NO: 1, and corresponding to a promoter.

9. Nucleic acid according to any one of claims 1 to 8, characterized in that it comprises all or part of the nucleotide sequence delimited by the nucleotide located at the position 1303 and the nucleotide located at the position 1965 of the nucleotide sequence represented by SEQ ID NO: 1, and coding for a protein of 221 amino acids represented by SEQ ID NO: 3.

10. Nucleic acid according to claim 9, characterized in that it comprises also all or part of the nucleotide sequence delimited by the nucleotide located at the position 1286 and the nucleotide located at the position 1302 of the nucleotide sequence represented by SEQ ID NO: 1, and more particularly the sequence TAAGG at positions 1286-1290, corresponding to a potential ribosome binding site.

11. Nucleic acid according to claim 9 or claim 10, characterized in that it comprises also all or part of the nucleotide sequence delimited by the nucleotide located at the position 1217 and the nucleotide located at the position 1285 of the nucleotide sequence represented by SEQ ID NO: 1, and more partcularly the sequences at positions 1219-1232 and positions 1263-1269, corresponding to a promoter.

12. Nucleic acid according to any one of claims 1 to 11, characterized in that it comprises all or part of the nucleotide sequence delimited by the nucleotide located at the position 2000 and the nucleotide located at the position 2437 of the nucleotide sequence represented by SEQ ID NO: 1, and coding for a protein of 146 amino acids represented by SEQ ID NO: 4.

13. Nucleic acid according to any one of claims 1 to 12, characterized in that it comprises all or part of the nucleotide sequence delimited by the nucleotide located at the position 2448 and the nucleotide located at the position 2475 of the nucleotide sequence represented by SEQ ID NO: 1, corresponding to a potential translational promoter.

14. Nucleic acid according to claim 1, characterized in that it comprises all or part of the nucleotide sequence delimited by the nucleotide located at the position 781 and the nucleotide located at the position 2423 of the nucleotide sequence represented by SEQ ID NO: 1.

15. Protein comprising the 56 amino acids sequence represented by SEQ ID NO: 2, and coded by the nucleic acid according to claim 6, this protein being able to allow the replication of a vector, the genome of which codes for such a protein, in a cellular host, or fragments or muteins (which differ from said protein by addition and/or substitution and/or suppression of one or several amino acids) thereof, provided that said fragments or muteins are still able to allow the replication of said vector.

16. Protein comprising the 221 amino acids sequence represented by SEQ ID NO: 3, and coded by the nucleic acid according to claim 9, this protein being able to allow the replication of a vector, the genome of which codes for such a protein, in a cellular host, or fragments or muteins (which differ from said protein by addition and/or substitution and/or suppression of one or several amino acids) thereof, provided that said fragments or muteins are still able to allow the replication of said vector.

17. Protein comprising the 146 amino acids sequence represented by SEQ ID NO: 4, and coded by the nucleic acid according to claim 12, this protein being able to allow the replication of a vector, the genome of which codes for such a protein, in a cellular host, or fragments or muteins (which differ from said protein by addition and/or substitution and/or suppression of one or several amino acids) thereof, provided that said fragments or muteins are still able to allow the replication of said vector.

18. Nucleic acids characterized in that they code for all or part of a protein according to claims 15 to 17.

19. Vector, such as plasmid, susceptible to replicate in a cellular host, characterized in that its genome comprises a nucleic acid according to any one of claims 1 to 14 or 18.

20. Vector according to claim 19, characterized in that it corresponds to plasmids pJK355, represented by SEQ ID NO: 5, and pJK356, represented by SEQ ID NO: 6, used for the transformation of the strain LK1 of *Lactobacillus casei*, these strains thus transformed being deposited at the Deutsche Sammlung von Mikroorganismen (DSM), on May 27th 1993, under the number 8322 and the number 8323, respectively.

21. Vector according to claim 19 or 20, characterized in that it contains in one (or several) of its site(s) non essential for its replication one (or several) heterologous sequence(s) coding for a predetermined polypeptide.

22. Vector according to claim 21, characterized in that said predetermined polypeptide is chosen among polypeptides produced by species and strains susceptible to be used as starter organisms in fermentation processes, and more particularly lactic acid bacteria, such as *Lactococcus, Streptococcus* and *Lactobacillus*, susceptible to produce aminopeptidase such as X-Prolyl-dipeptidyl-aminopeptidase (PepX) from *Lactobacillus delbrückii* ssp. *lactis or* polypeptides derived thereof.

23. Cellular host, such as lactobacilli, transformed by a vector according to any one of claims 19 to 22.

24. Process for the production of a predetermined polypeptide characterized in that it comprises the following steps:
- the culture in an appropriate medium of a cellular host according to claim 23,
- the recovery of the predetermined polypeptide produced by the above said cellular host from the above said culture medium,
- the purification of the polypeptide thus obtained.

25. Fermentation process characterized in that it comprises the culture in an appropriate medium, of a cellular host according to claim 23.

26. Process for the preparation of fermented foodstuff, and more particularly of sausage or cheese, which comprises a step of treatment of food material to be fermented, such as milk, with an appropriate amount of at least one of the transformed cellular host according to claim 23.

27. Process according to claim 26, characterized in that it also comprises a step of treatment of food material to be fermented, such as milk, with other species and strains susceptible to be used as starter organisms in fermentation processes, and more particularly lactic acid bacteria, such as *Lactococcus, Streptococcus* and *Lactobacillus*, susceptible to produce an aminopeptidase useful in fermentation processes, such as PepX protein.

28. Foodstuff, and more particularly cheese or sausage, such as obtained by the process according to any one of claims 25 to 27.
